# EUROPEAN PATENT APPLICATION

(11) **EP 2 871 235 A1**
(43) Date of publication of application: **13.05.2015**
(21) Application number: 13306526.8
(22) Date of filing: 07.11.2013
(51) Int. Cl.: C12N 9/12, C12N 15/81, C12N 15/62

(54) **New methods to produce active hTERT**

(71) Applicant: Centre National de la Recherche Scientifique (CNRS), 75016 Paris (FR); INSTITUT NATIONAL DE LA SANTE ET DE LA RECHERCHE MEDICALE (INSERM), 75013 Paris (FR); UNIVERSITE PARIS DESCARTES, 75006 Paris (FR)
(72) Inventor: Kellermann, Guillaume, 94200 Ivry (FR); Lahuna, Olivier, 94800 Villejuif (FR); Bombard, Sophie, 91600 Savigny S/Orge (FR); Segal-Bendirdjian, Evelyne, 91420 Morangis (FR)
(74) Representative: Regimbeau

(57) **Abstract**

The present invention relates to a method to reproducibly produce large amounts of soluble and active hTERT by expressing this enzyme in yeast cells. High yield of recombinant active hTERT has been obtained by controlling specific parameters of the purification process. The hTERT protein thus obtained displays telomerase activity with *in vitro* transcribed hTR, opening the way to high throughput chemical screening of telomerase assembly inhibitors and therapeutic applications.

## Description

### BACKGROUND OF THE INVENTION

Telomeres are structures located at the ends of eukaryotic chromosomes containing noncoding repeated DNA sequences (TTAGGG in humans). These regions progressively shorten in the successive rounds of cell division, causing the loss of essential genetic information and eventually the death of the cell. The presence of telomeric regions therefore hinders the loss of DNA from chromosome ends, resulting in protection against the phenomenon of cellular senescence and aging.

The maintenance of telomeres is a function of a telomere-specific DNA polymerase known as *telomerase.* Telomerase is a reverse transcriptase that carries its own RNA molecule (hTR), which is used as a template for the addition of multiple TTAGGG repeats to the 3'-end of the G-rich strand of telomeres. Telomerase therefore contains two essential components, a protein core having reverse transcriptase (RT) activity and an RNA molecule (hTR). The core domain of human telomerase is also called the "telomerase catalytic protein subunit" or "hTERT" (for human telomerase reverse transcriptase). This subunit is a 127 kDa polypeptide containing three regions: i) the catalytic RT domain, ii) a telomerase-specific N-terminal domain that has been implicated in telomerase activity, binding to the RNA subunit and multimerization, and iii) a C-terminal extension that presumably has a role in promoting enzyme processivity (Autexier C. et al, Annu. Rev. Biochem. 2006). In cells, hTERT and its template RNA are part of a larger complex that includes a number of other proteins, including TLP1 (Nakayama J. et al., Cell 1997), hsp90, hsp23 (Holt S.E. et al., Genes Dev. 1999), and dyskerin (Mitchell J.R. et al. Nature 1999).

It is now widely accepted that telomerase activity promotes the immortality of many cancer types. Consequently, inhibiting the hTERT catalytic component would be expected to restore the telomere shortening process and ultimately cause cancer cell death, without affecting normal somatic cells, since these cells do not express telomerase. Thus, inhibitors of hTERT enzymatic activity or telomerase assembly are held as promising tools for cancer treatment (Zhang X. et al, Genes Dev. 1999).

Identifying efficient telomerase inhibitors however requires the prior *in vitro* reconstitution of a processive telomerase complex and, in particular, of an active hTERT catalytic subunit, in order to implement conclusive screening assays.

Moreover, generating high amounts of active form of recombinant hTERT may be of primary importance to develop vaccines aimed at eliciting anti-telomerase immune responses that could be used in immunotherapeutic protocols for cancer-suffering patients.

Finally, recombinant hTERT could be advantageously used in pharmaceutical compositions in order to treat diseases and conditions characterized by the absence of human telomerase activity, such as diseases associated with cell senescence (particularly diseases of aging) and infertility.

There is therefore a great need in obtaining high amounts of a recombinant hTERT protein which is soluble and active. However, so far, no satisfactory expression and purification protocol has been developped.

In fact, purifying large amounts of properly folded and enzymatically active recombinant hTERT is viewed as an exceptionally difficult problem, as various attempts to produce this protein, using different expression and purification systems, gave only limited results. Full length hTERT cannot be overexpressed (Holt S.E. et al., Genes Dev. 1999) or has been reported to be unsoluble (Masutomi K. et al. J. Biol. Chem. 2000; Mikuni O. et al. BBRC 2002 ; Wu C.K. et al. Protein Expr. Purif. 2007). Solubilization of the enzyme using the MEGA-9 detergent (as proposed by Masutomi K. et al. J. Biol. Chem. 2000) is not reproducible (Wu C.K. et al. Protein Expr. Purif. 2007). Moreover, the purification of active hTERT by means of an affinity tag is poorly efficient: His-hTERT fusions have been expressed, but no successful purification has been reported with His Tag (Bachand F. et al, JBC 1999 and Wenz et al. EMBO 2001). As an alternative to purification, secreting hTERT by insect cells is unsuccessful because the enzyme remains bound to the endoplasmic reticulum (Wu et al, Protein Expr. Purif. 2007). Finally, hTERT purified using glutathione- or heparin-sepharose has been reported to lose its telomerase reconstitution capability after column purification (Bachand F et al, JBC 1999 and Mizuno H. et al, J. Biochem., 2007). It therefore seems that the tagged-hTERT protein cannot be easily produced to prepare recombinant telomerase. In view of all these unsuccessful attempts, it was concluded that human telomerase "is challenging to purify and cannot be prepared in large quantities" (Alves D. et al, Nat. Chem. Biol. 2008).

The present inventors however herein show that it is actually possible to reproducibly produce large amounts of soluble and active hTERT by expressing this enzyme in yeast cells such as, e.g., *Pichia pastoris* cells. High yield of recombinant active hTERT can be further obtained by controlling specific parameters of the purification process, as detailed below. The hTERT protein thus obtained displays telomerase activity with *in vitro* transcribed hTR, opening the way to high throughput chemical screening of telomerase assembly inhibitors and therapeutic applications.

### FIGURE LEGENDS

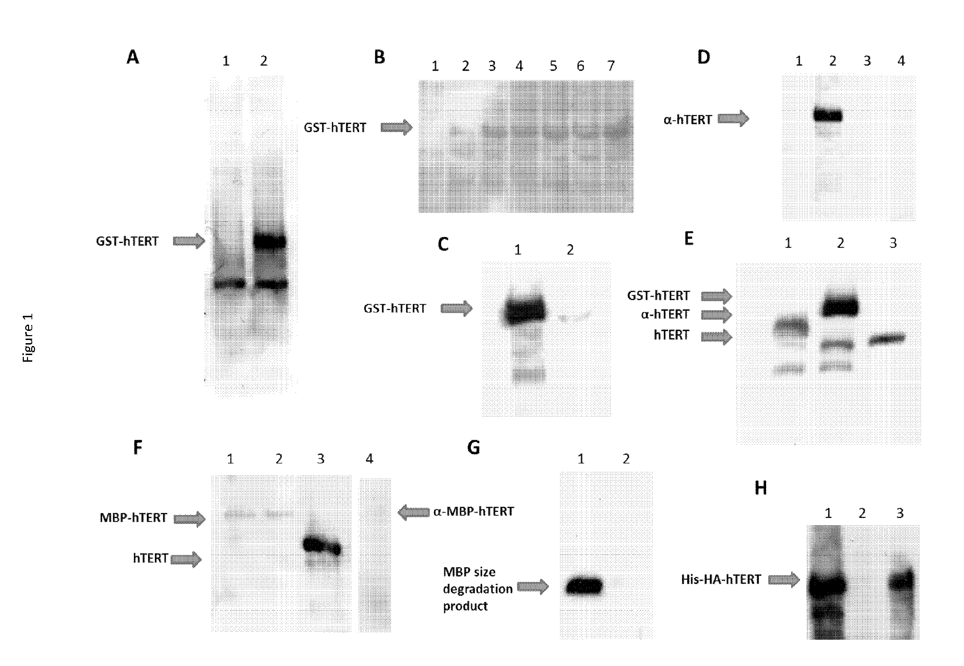

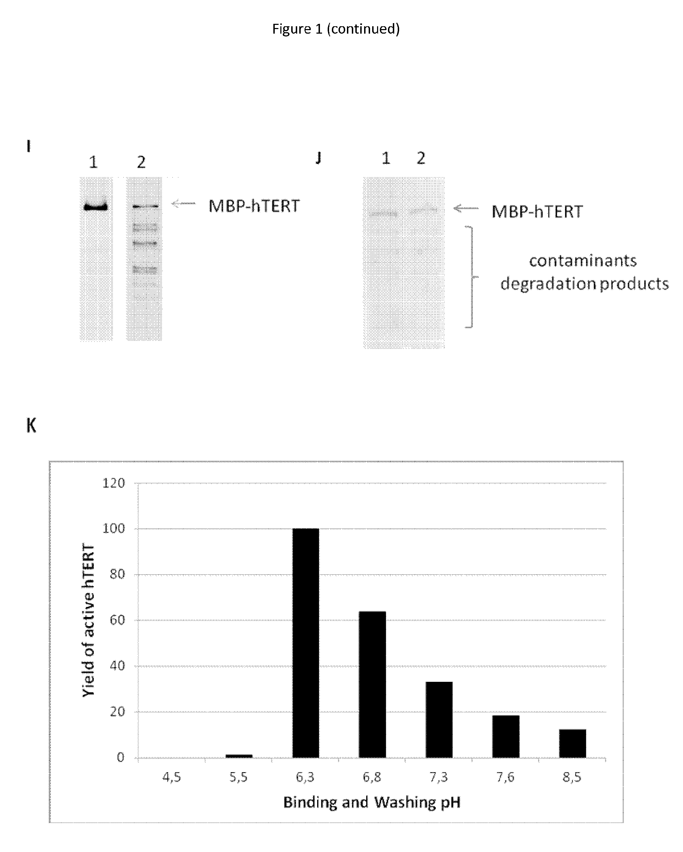

**Figure 1****: hTERT can be expressed using the PGAPZ vector, but cannot be purified unless the specific MBP tag is used.**
   **A) GST-hTERT (155kDa) is expressed intracellulary using PGAPZ vector.** A western-blot was performed on the cell extract from wild-type *Pichia pastoris* (lane 1) or on the cell extract from *Pichia pastoris* cells expressing GST-hTERT (lane 2) using an anti-GST antibody (Sigma). **B) Zeocin-resistant clones express GST-hTERT. A** western-blot was performed on the cell extract from wild-type *Pichia pastoris* (lane 1) or on cell extracts of different *Pichia pastoris* clones transformed to express GST-hTERT (lanes 2-7) using an anti-GST antibody (Sigma). **C) GST-hTERT show poor affinity for glutathione-sepharose** (lane 1: flow; lane 2: bound-fraction). D) **α-hTERT cannot be secreted.** Western Blot with anti-hTERT antibody on intracellular fraction (Lane 1-2) or extracellular fractions (lane 3-4) of wild-type *Pichia pastoris* cells (lanes 1,3) or α-hTERT transformed yeast (Lanes 2,4) corresponding to hTERT fused to the secretion signal of *Saccharomyces* s. alpha factor. **E) Comparison of the hTERT expression level containing different tags.** The soluble intracellular content of yeast was analyzed by Western Blot with the anti-hTERT antibody on *Pichia pastoris* cells transformed with α-hTERT (lane 1), GST-hTERT (lane 2), and untagged hTERT (lane 3, 127 kDa). **F) MBP-hTERT and α-MBP-hTERT are expressed at lower level in *Pichia pastoris* cells.** Western Blot on cell extracts of *Pichia pastoris* cells transformed with MBP-hTERT (lanes 1 and 2), hTERT (lane 3) and α-MBP-hTERT (Lane 4) with an anti-hTERT antibody. **G) MBP-hTERT and α-MBP-hTERT protein are not detected with the methanol based expression system.** Western Blot with an anti-HA antibody on *Pichia* cells expressing MBP-hTERT under the control of the AOX1 promoter (pPIC3.5K Vector) (lane 1) or on Pichia cells expressing α-MBP-hTERT(lane 2). **H) The N-terminal part of hTERT is accessible.** Western Blot after immunoprecipitation of His-HA-hTERT from a total cell lysate of *Pichia pastoris* cells expressing His-HA-hTERT using an anti-HA tag antibody (lane 3). Controls: total cell lysate from His-HA-hTERT expressing yeast (lane 1) and immunoprecipitation with A+G beads only (lane 2). **I) Low intracellular pH leads to protein degradation** When hTERT is purified in the conditions of the invention (at intracellular pH 6,3) it is not degraded (lane 1), while it is significantly degraded when the intracellular pH is 5,5 (lane2). **J) Protease Inhibitor and detergents are not required.** Standard purification was performed (lane 1) and compared to a purification performed with the addition of a protease inhibitor cocktail (Roche) and 1% triton, in extraction and washes solutions (lane 2), which did not increased the yield and improved only very slightly the purity. **K) Best purification occurs with slightly-acid/neutral pH.** The pH of the extract was adjusted to different pH before column binding and the washing steps were then performed with the corresponding pH for each condition.

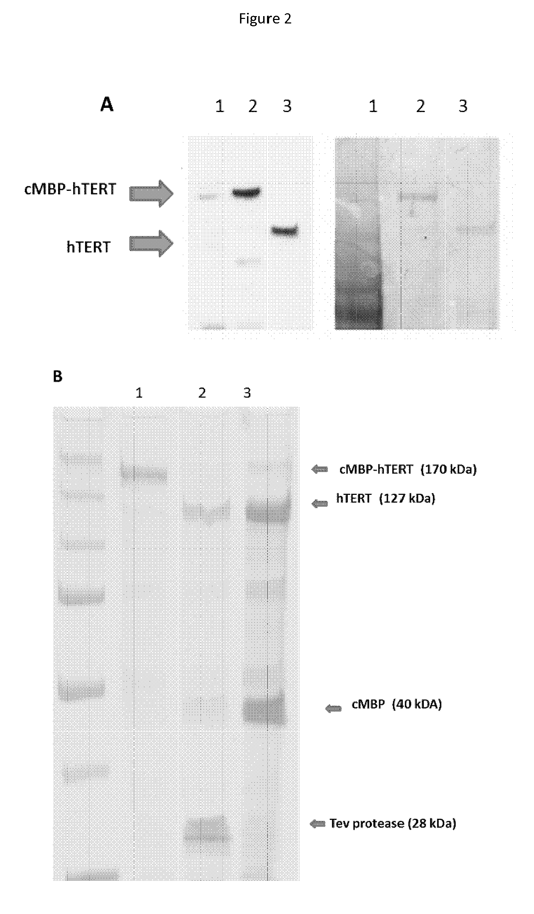

**Figure 2****: Purification of cMBP-hTERT in *Pichia pastoris.* A) cMBP-hTERT purification and cleavage.** cMBP-hTERT was purified using amylose-sepharose. The purified protein was monitored by both Western Blot (left) and Coomassie Brilliant Blue (right). Lane 1: the cell extract, lane 2: cMBP-hTERT eluted from amylose-beads, and lane 3: the protein after cleavage of the MBP tag by TEV protease. **B) cMBP-hTERT purity and MBP TAG removal efficiency.** Lane 1: cMBP-hTERT after the elution from the amylose column. Lane 2: cMBP-hTERT cleaved by an excess of His-TEV protease for one hour at room temperature. Lane 3: TEV protease was removed by nickel-column rebinding, and the sample reconcentrated to evaluate more accurately the purity level and the efficiency of the cleavage by TEV protease.

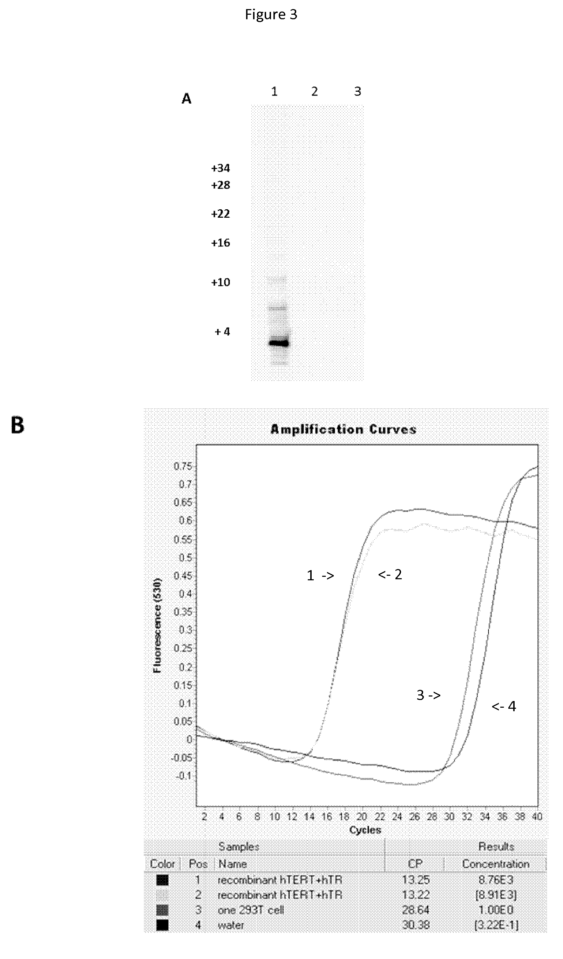

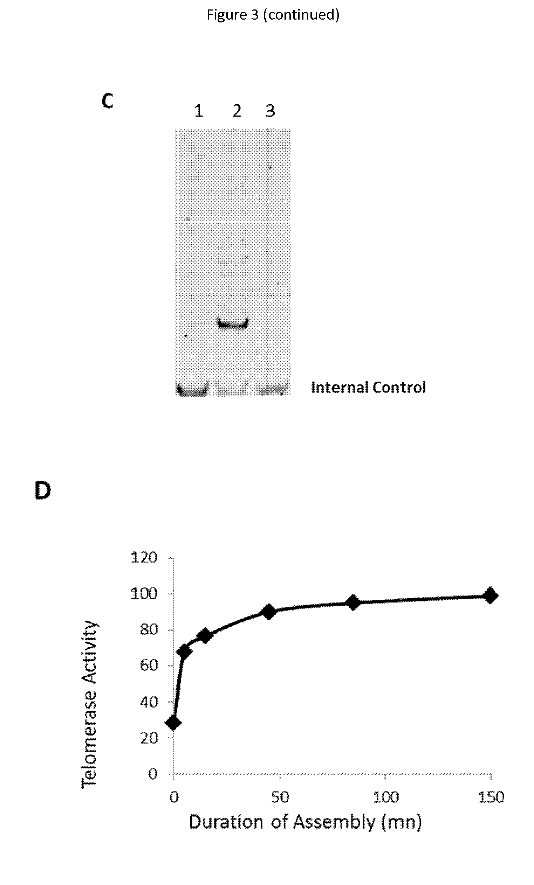

**Figure 3****: Reconstitution of telomerase activity.** Telomerase activity was reconstituted by adding 500ng of *in vitro* transcribed hTR to 100ng of purified cMBP-hTERT and detected by several methods. **A) Telomerase Direct Assay** with cMBP-hTERT and hTR (lane 1), cMBP-hTERT alone (lane 2) or hTR alone (lane 3). B) **qTRAP.** Quantitative measurement of telomerase activity with a LightCycler II (Roche) showed that the reconstituted activity is around 9 000 fold higher (1, 2) than the one found in one 293T cell (3). **C) TRAP** (Telomeric repeat amplification protocol) with hTR alone (lane 1), cMBP-hTERT and hTR (lane 2) and MBP-hTERT alone (lane 3). **D) Kinetic of telomerase reconstitution** measured by qTRAP.

### DETAILED DESCRIPTION OF THE INVENTION

Successful expression of the hTERT subunit of telomerase has been already reported, for example in insect cells (Masutomi K. et al. J. Biol. Chem. 2000; Wenz C. et al. EMBO 2001; Mikuni O. et al. BBRC 2002, and Wu C.K. et al, Protein Expr. Purif. 2007) or in yeast cells (Bachand F. et al, JBC 1999). However, all these methods required the use of protease inhibitors, which significantly impacts the costs of these operations. They are therefore not appropriate to obtain large amounts of the hTERT enzyme.

The present invention relates to a method for producing reproducibly high amounts of soluble and active hTERT at low cost. The method of the invention requires the use of recombinant vectors (e.g., integrative plasmids) that are introduced in yeast cells. Importantly, cell lysis and purification of hTERT are performed without requiring protease inhibitors. Using commercial lysis buffers is also not recommended, as pure water was shown to give the best results. The method of the invention can therefore be performed in laboratories having conventional facilities, at low cost.

The present inventors disclose the expression of correctly folded hTERT in a yeast system, and the reproducible recovery of large amounts of this enzyme by means of a tightly regulated purification process. Importantly, and in contrast with the protocols disclosed in the prior art, the present expression and purification processes enable to recover enzymatically active hTERT, after purification. Furthermore, the enzyme recovered by means of the method of the invention is soluble, and can efficiently be used in screening methods for identifying potent and specific inhibitors of human telomerase assembly and/or activity.

The inventors tested and compared numerous experimental conditions in order to identify the best conditions for each step of the expression and purification processes. They consequently identified an optimized and reproducible method to produce *in vitro* the hTERT subunit of telomerase, what was thought to be a great challenge.

More precisely, the method of the invention contains at least four distinct steps:
a) Providing a yeast cell comprising a nucleotide vector encoding the hTERT protein tagged with an appropriate tag,
b) Growing said cells in specific conditions, so that the recombinant hTERT protein is produced intracellularly by the yeast cells,
c) Preparing a crude protein extract of the said yeast cells, in specific conditions,
d) Purifying the hTERT protein by means of an appropriate affinity purification protocol.

Even more precisely, said appropriate tag is the maltose-binding protein (MBP) tag deleted of its N-terminal periplasmic targeting signal (cMBP). In fact, the present inventors have shown that large amounts of active hTERT can be recovered from yeast when hTERT is bound to said tag, and that expression of the cMBP-hTERT fusion protein is advantageously enhanced when it is operatively linked to a constitutive promoter which is functional in yeast (e.g., the GAPDH promoter).

The inventors have also demonstrated that the growth of the cMBP-hTERT-expressing yeast cells has to be precisely monitored until the stationary phase is reached and that the media in which the yeast cells are cultured is of primary importance.

For the extraction of cMBP-hTERT fusion protein, no complex lysis buffer is required as mechanical shearing could be performed in cold pure water, that advantageously does not contain any protease inhibitor. The purification step is then preferably performed by using binding and washing buffers whose pH is comprised between 6.0 and 7.5, more preferably between 6.3 and 7.0. The use of binding and washing buffers whose pH is below 7.6, allows an enhancement of the yield and of the specific activity of the purified hTERT.

By reproducing these experimental conditions, the skilled person will be able to produce at low cost high amounts of the hTERT enzyme in an active form. Typically, it is possible to recover 0,3 mg of the active and soluble hTERT enzyme by liter of yeast cells grown in shake flask.

In a first aspect, the present invention relates to a method to produce soluble and active human telomerase reverse transcriptase (hTERT) protein, comprising the steps of:
a) Growing yeast cells comprising a nucleotide vector, said vector containing a constitutive promoter operatively linked to a nucleotide sequence encoding a fusion protein containing:
   - a maltose-binding protein tag deleted of its N-terminal periplasmic targeting signal (cMBP), and
   - a human telomerase reverse transcriptase (hTERT) protein,
b) Preparing a crude protein extract of the cells of step a),
c) Adjusting, if necessary, the pH of said extract to a pH comprised between 6.0 and 7.5,
d) Purifying the fusion protein cMBP-hTERT by means of an amylose-coupled solid support.

Preferably, preparing a crude protein extract of the cells of step a), is performed before yeast cells intracellular pH reaches a value below 5.8 and more preferably before yeast cells intracellular pH reaches a value below 6.3.

Preferably, in step c), the pH of said extract is adjusted to a pH comprised between 6.3 and 7.0. This step is not necessary if said crude protein extract has already a pH comprised between 6.3 and 7.0 after step b).

Telomerase reverse transcriptase (abbreviated to TERT, or hTERT in humans) is the catalytic subunit of the telomerase enzyme. This subunit, together with the telomerase RNA component (hTR), is the most important component of the telomerase complex (Weinrich SL. et al, Nat. Genet. 1997). Specifically, hTERT is responsible for catalyzing the addition of a TTAGGG sequence (SEQ ID NO:5) at the end of each of the chromosome telomeres. This addition of DNA repeats prevents degradation of the chromosomal ends following multiple rounds of replication (Poole JC et al, Gene 2001). hTERT has for example the SEQ ID NO:1 (isoform 1, Genbank accession number: NP_937983.2) . This enzyme is a RNA-dependent DNA polymerase displaying a reverse transcriptase (RT) activity, i.e., it is able to generate complementary DNA (cDNA) from an RNA template.

The term "human telomerase reverse transcriptase (hTERT) protein" herein refers to the hTERT protein of SEQ ID NO:1 as well as variants thereof. In the context of the invention, "hTERT variants" are defined as sharing at least 75%, preferably at least 80% and more preferably at least 90% sequence identity with SEQ ID NO:1 and having the same telomerase catalytic activity as the enzyme of SEQ ID NO:1.

Usually, hTERT variants have a molecular weight of between 100 kDa and 150 kDa, preferably between 100 kDa and 130 kDa. They can differ from the hTERT protein of SEQ ID NO : 1 by internal deletions, insertions, or conservative substitutions of amino acid residues. Examples of preferred variants are disclosed in EP 0 841 396. Other examples of catalytically active hTERT protein variants have been provided in the literature. Mutants lacking the linker regions L1 and L2 which are dispensable for telomerase activity are described in Armbruster et al , Mol. Cell. Biol. 2001. Also, Banik SSR et al (Mol. Cell. Biol. 2002) have described mutants presenting mutations between the regions E-I to E-IV, which tolerate substitution without affecting the catalytic activity of hTERT.

The term "sequence identity" refers to the degree of identity or correspondence between amino acid sequences. In the context of the invention, two amino acid sequences have at least 75%, preferably at least 80% and more preferably at least 90% identity if at least 75%, preferably at least 80% and more preferably at least 90% respectively of their amino acids are identical. Preferably the identity of amino polypeptide sequences is identified by using the global algorithm of Needleman and Wunsch (J.Mol.Biol. 1970).

As used herein, a hTERT protein variant has "the same telomerase catalytic activity" as the hTERT protein of SEQ ID NO:1 if said hTERT protein variant has the same Reverse Transcriptase activity as the enzyme of SEQ ID NO:1. More precisely, these variants should be capable of extending a DNA primer or a chromosome telomere by adding as many repeats of the sequence TTAGGG (SEQ ID NO:5) as the enzyme of SEQ ID NO:1 does. Methods to measure the Reverse Transcriptase activity are well-known in the art. Some of them are disclosed below.

Preferably, when used in the present application, the term "hTERT protein" designates the hTERT protein of SEQ ID NO:1 itself.

The nucleotide vector used in the method of the invention contains a nucleotide sequence encoding a human telomerase reverse transcriptase (hTERT) protein. It will be appreciated that, as a result of the degeneration of the genetic code, the said nucleotide sequence need not to have the sequence of the naturally occurring hTERT gene (NM_198253.2, SEQ ID NO: 2). In fact, a multitude of polynucleotides encodes an hTERT protein having an amino acid sequence of SEQ ID NO: 1 or a variant thereof. Reagents and methods for cloning nucleotide encoding the hTERT protein or variants thereof are for example disclosed in EP 0841396. In particular, it is advantageous to use a nucleotide sequence encoding hTERT which has been optimized for yeast expression.

As used herein, the term "nucleotide vector" means a vehicle by which a DNA or a RNA sequence of a foreign gene can be introduced into a host cell. Nucleotide vectors may include for example plasmids, phages, and viruses. Three types of vectors can be used in yeast: integrative vector plasmids (Ylp), episomal plasmids (YEp), and centromeric plasmids (YCp). Suitable vectors for expression in yeast include, but are not limited to pYepsec1, pMFa, pJRY88, pYES2 (Invitrogen Corporation, San Diego, Calif.), PGAPZ (Invitrogen) and pTEF-MF (Dualsystems Biotech Product) pKLAC2 (New England Biolabs). Preferably, the nucleotide vector of the invention is an integrative plasmid, that is, a plasmid which relies on integration into the host chromosome for survival and replication. More preferably, this nucleotide vector is the integrative plasmid PGAPZ (Invitrogen).

A sequence "encoding" an expression product such as a RNA or an enzyme, is a nucleotide sequence that, when expressed, results in the production of said RNA or said enzyme.

In the vector of the invention, the open reading frame encoding the hTERT protein is operatively linked to a constitutive promoter. A coding sequence is "operatively linked to" a promoter sequence controlling its expression when RNA polymerase transcribes the said coding sequence into a RNA, which is then translated into a protein.

A "promoter" is a sequence of nucleotides from which transcription may be initiated (i.e., in the 3' direction on the sense strand of double- stranded DNA). Within the promoter sequence will be found a transcription initiation site (conveniently found, for example, by mapping with nuclease S1), as well as protein binding domains (consensus sequences) responsible for the binding of RNA polymerase. Promoters can be constitutive (that is, they are active in all circumstances in a yeast cell and allow continual transcription of its operatively associated gene) or inducible (that is, their activity is induced by the presence or absence of defined biotic or abiotic factors).

Yeast cells which can be used in the method of the invention are preferably selected from the group consisting of: *Saccharomyces cerevisiae, Schizosaccharomyces pombe, Kluyveromyces lactis* and *Hansenula polymorpha,* as well as methylotropic yeasts like *Pichia pastoris* and *Pichia methanolica.* In a preferred embodiment, the yeast cells used in the method of the invention are *Pichia pastoris* cells.

Promoters which are preferably used to control the expression of the gene of the present invention are those that act constitutively in yeast cells. Several constitutive yeast promoters are available for protein expression in yeast host cells. These include for example: the pCYC promoter, the pAdh promoter, the pSte5 promoter, the yeast ADH1 promoter, the cyc100 minimal promoter, the cyc70 minimal promoter, the cyc43 minimal promoter, the cyc28 minimal promoter, the cyc16 minimal promoter, the pPGK1 promoter, the CLB1 promoter, and the glyceraldehyde-3-phosphate dehydrogenase (GAP or GAPDH) promoter (the sequence of these promoters is disclosed for example in http://parts.igem.org/Promoters/Catalog/Yeast/Constitutive).

In a preferred embodiment, the nucleotide vector of the invention contains the GAPDH promoter. This promoter is indeed constitutively functional in *Pichia pastoris* yeast cells. In a more preferred embodiment, said GAPDH promoter has the sequence SEQ ID NO:10.

The nucleotide vector of the invention also necessarily contains a nucleotide sequence encoding a maltose-binding protein (MBP) tag which is deleted of its N-terminal periplasmic targeting signal. The Maltose-Binding Protein (MBP) is a part of the maltose/maltodextrin system of *Escherichia coli,* which is responsible for the uptake and efficient catabolism of maltodextrins. It is a complex transport system involving many proteins. Its N-terminal part contains a periplasmic targeting signal, also called "periplasmic signal peptide" (SEQ ID NO:7). This signal peptide is involved in the export of the MBP protein into the periplasm of bacterial cells.

Fusion with the full-length MBP tag (SEQ ID NO:6) is often used to increase the solubility of recombinant proteins expressed in *E. coli.* In addition, MBP can be used as an affinity tag for purification of recombinant proteins. Typically, fusion proteins containing MBP bind to amylose columns while all other proteins flow through. These fusion proteins can be purified by eluting the column with maltose. In the present invention, MBP is used as a tag for favoring the purification of hTERT.

Contrary to a previous report (Wu et al, Protein Expr. Purif. 2007), the inventors have shown that, the yield of hTERT purification is enhanced by impairing the extracellular export of the hTERT protein. This is achieved by removing the MBP N-terminal periplasmic targeting signal in the fusion protein MBP-hTERT. The MBP tag deleted of its N-terminal periplasmic targeting signal will be hereafter referred to as "cMBP". cMBP has preferably the SEQ ID NO:8.

In a preferred embodiment, the nucleotide vector of the invention does not contain any secretion signal which is functional in yeast, so that the hTERT protein will remain intracellular.

Thus, in a preferred embodiment, the hTERT protein is tagged with the maltose-binding protein tag of sequence SEQ ID NO:8 (corresponding to the maltose-binding protein tag deleted of its N-terminal periplasmic targeting signal). This cMBP tag is for example encoded by the nucleotide sequence SEQ ID NO:9.

In a preferred embodiment of the invention, the nucleotide sequence encoding the hTERT protein is located in 5' of the nucleotide sequence encoding the cMBP tag. In another embodiment of the invention, the nucleotide sequence encoding the hTERT protein is located in 3' of the nucleotide sequence encoding the cMBP tag. Consequently, in the fusion protein of the invention, the cMBP tag can be located at the C-terminal or at the N-terminal end of the hTERT enzyme. Preferably the cMBP tag is located at the N-terminal end of the hTERT enzyme.

The fusion protein of the invention contains the hTERT enzyme which is directly or indirectly linked to the cMBP tag. The two polypeptides may be in particular separated by a spacing sequence (or "spacer") that impairs steric hindrance between them. In a preferred embodiment, the fusion protein of the invention may therefore contain a spacer located between the cMBP tag and the hTERT protein. In this embodiment, the nucleotide vector of the invention consequently contains a spacer-encoding nucleotide sequence between the nucleotide sequences encoding for hTERT and the cMBP. This spacer has for example the SEQ ID NO:12 or SEQ ID NO:13.

Once the fusion protein cMBP-hTERT is obtained in purified form, it may be advantageous to separate the protein of interest hTERT from the cMBP tag. This separation can be achieved by means of a specific protease if the fusion protein of the invention contains a protease cleavage site located between the cMBP tag and the hTERT protein.

Advantageously, the nucleotide vector of the invention may further contain a nucleotide sequence encoding a known protease cleavage site. This nucleotide sequence is preferably located between the sequence encoding the maltose-binding protein tag and the sequence encoding the hTERT protein.

Protease cleavage sites are well-known in the art. They are amino acid sequences which are recognized by at least one protease enzyme (for example a serine protease or a cysteine protease, among others). An example of a peptidic cleavage site is the enterokinase cleavage site of SEQ ID NO:16 (AspAspAspAspLys/Asp). The enterokinase is a serine protease enzyme (EC 3.4.21.9) which is known to convert inactive trypsinogen into active trypsin by cleavage at the C-terminal end of the sequence: Val--(Asp)₄--Lys--Ile--Val∼ (trypsinogen) → Val--(Asp)₄--Lys (hexapeptide) + Ile--Val~ (trypsin). Enterokinase cleaves after Lysine if the Lys is preceded by four Asp and not followed by a Proline residue. Another useful protease cleavage site is the cleavage site of the so-called "TEV protease", having the amino acid sequence SEQ ID NO:14 or SEQ ID NO: 15 (Glu Asn Leu Tyr Phe Gin Gly or Ser). TEV protease is the common name for the 27 kDa catalytic domain of the nuclear inclusion a protein encoded by the tobacco etch virus. It is commercially available (Invitrogen).

In a preferred embodiment, the vector of the invention contains a sequence encoding the TEV protease cleavage site (SEQ ID NO:14 or SEQ ID NO:15) located between the sequence encoding the maltose-binding protein tag and the sequence encoding the hTERT protein.

As used herein, the term "cMBP-hTERT" is used interchangeably with the expression "fusion protein of the invention". They designate a fusion protein containing the hTERT polypeptide sequence or a variant thereof, and a maltose-binding protein tag deleted of its N-terminal periplasmic targeting signal, in all their possible spatial orientation (hTERT-cMBP or cMBP-hTERT), the two moieties of this fusion protein being optionally separated a spacer and/or a protease cleavage site.

In a more preferred embodiment, the nucleotide vector used in the method of the invention comprises a constitutive promoter which is functional in yeast, such as the GAPDH promoter, which is operatively linked to a nucleotide sequence encoding the fusion protein cMBP-TEV-hTERT.

In a particular embodiment, the nucleotide vector is the integrative plasmid PGAPZ (Invitrogen) containing the GAPDH promoter which is operatively associated with a nucleotide sequence encoding the fusion protein **cMBP-hTERT** or cMBP-TEV-hTERT (SEQ ID NO:17) (or hTERT-cMBP, or hTERT-TEV-cMBP). This vector has the sequence SEQ ID NO:18.

The expression vector of the invention may be introduced into the yeast cells of the invention by any method known in the art. Preferably, the said vector is transformed into the yeast cells. Several transformation methods are known to the skilled person. They include lithium acetate based transformation, spheroplasting, electroporation, etc.

The term "transformation" herein means the introduction of a heterologous nucleic acid encoding a defined protein into a yeast host cell so that said cell will express the protein encoded by the introduced nucleic acid. A host cell that receives and expresses introduced nucleic acid has been "transformed".

The transformed yeast cells are subsequently grown so that the expression of the fusion protein is allowed. Media that are conventionally used for growing yeast cells are herein recommended. The skilled person knows well these media, that have been extensively described.

In a preferred embodiment, the transformed yeast cells used for expression experiments have been previously grown on an agarose petri dish (containing e.g., agar 1,5%, 1% yeast extract, 2% peptone, 0.2% Yeast Nitrogen Base with ammonium sulfate, 2% Dextrose).. More preferably, this preculture step lasts from one to two days TInterestingly, this preculture step enhances the specific activity and the stability of the purified enzyme, and also decreases the level of contaminating RNA.

In an another preferred embodiment, the transformed yeast cells are grown in a nutrient medium containing at least 0.5%, more preferably 1%, and even more preferably at least 2% of yeast extract. The said nutrient medium may also contain a carbon source (such as glucose) and salts (such as NaHPO₄) Glucose may be present in said nutrient medium in an amount of about 2 to 8 %, preferably at 4%. NaHPO₄ may be present in said nutrient media in an amount of about 10 to 300mM, preferably of about 50 to 200mM, and more preferably at 100mM. The initial pH of the nutrient culture medium is preferably comprised between 6.0 and 7.0. Furthermore, the yeast cells are preferably grown at a temperature comprised between 15°C and 35°C, preferably between 27°C and 30°C.

By growing the transformed yeast cells in these conditions, their intracellular pH slowly decreases. Yet, and importantly, the intracellular pH of the yeast cells, measured after mechanical shearing, should not decrease below the value of 5.8, preferably of 6.3. In other words, the yeast cells are grown in the nutrient media as long as their intracellular pH is superior or equal to about 5.8, preferably to about 6.3. Methods for measuring intracellular pH are well-known in the art (see, for a review Loiselle FB and Casey JR, Methods Mol. Biol. 2010). For example, intracellular pH can be measured by lysing the yeast cells in cold water at cold temperature (typically at 4°C) and by measuring the pH in the supernatant with a pH meter.

Of note, this critical pH value is reached soon after the end of the exponential growth, i.e., at the beginning of the stationary growth phase. In practice, it has been observed by the inventors that the critical intracellular pH values of 6.3 / 5.8 (measured after cell lysis) are reached when the optical density at 600nm (OD₆₀₀) of the yeast-containing culture is comprised between 11 and 16, preferably between 12 and 15 (as measured for example with a spectrophotometer Eppendorf).

During cell growth, the cMBP-hTERT fusion protein is expressed and accumulates within the yeast cells without being secreted. Advantageously, yeast cells are lysed so as to release the intracellular accumulated cMBP-hTERT fusion proteins. This lysis step b) should be performed before the intracellular pH of the transformed cells reaches 5.8, preferably 6.3. In a particular embodiment, this lysis step b) should be performed once the intracellular pH of the transformed cells reaches 6.3.

In a preferred embodiment, the yeast cells are lysed in step b) in a water-based solution. In a more preferred embodiment, said water-based solution is salt and detergent-free. In an even more preferred embodiment, said water-based solution is pure water. In a particularly preferred embodiment, said water-based solution does not contain any protease inhibitor. The inventors have indeed observed that, surprisingly, the hTERT protein is not sensitive to proteolytic degradation in the optimised culture conditions, and that protease inhibitors are not required. This renders the process of the invention less expensive than the purification protocols of the prior art.

The lysis step b) is preferably achieved at cold temperature, i.e., at a temperature comprised between 0°C and 10°C (typically at 4°C).

In a preferred embodiment, cell lysis is performed by any conventional means. It is for example favored by breaking mechanically the yeast cell walls using any physical means, such as a French press. Alternatively, dedicated glass beads may be added to the water-based lysis solution containing the yeast cells, said mixture being subsequently vigorously vortexed during e.g. 5 to 15 minutes, preferably 10 minutes. Cell fragments (such as cell debris and large organelles) and glass beads can be advantageously discarded by centrifugating the mixture at appropriate speed (e.g., 3000 g for 10 minutes, then optionally at 10 000g for 15 minutes). Cell lysis and centrifugation should be achieved at cold temperature, i.e., at a temperature comprised between 0°C and 10°C (typically at 4°C).

Obtention of the crude protein extract containing the cMBP-hTERT fusion protein can be performed by centrifugating the water-based solution containing the yeast cells at cold temperature. The "crude protein extract" herein corresponds to the fraction of the solution containing the intracellular proteins - among which the fusion protein cMBP-hTERT- and almost no fragments or debris of cell walls and large organelles.

The skilled person is fully aware of the appropriate centrifugation conditions that may be used to efficiently remove all the cell fragments (such as cell debris and large organelles) without altering the amount or the activity of the hTERT protein of interest. Centrifugation speed is typically comprised between 3,000 g and 20,000g. Non-compacted particles such as soluble proteins remain mostly in the liquid called "supernatant" and can be transferred in another tube thereby separating the proteins from the cell fragments. The supernatant is then used for further purification steps.

The method of the invention may further necessitate increasing the pH of the water-based solution containing the lysed cells so as to reach a pH comprised between 6.0 and 7.5, preferably to a pH comprised between 6.3 and 7.0 (step c). This pH increase is preferably achieved in the crude protein extract obtained after step b). However, it is also possible to increase the pH before the cell fragments are removed. This step is not necessary if said crude protein extract already has a pH comprised between 6.0 and 7.5, or more preferably comprised between 6.3 and 7.0.

The inventors indeed observed that maintaining the pH of the hTERT-containing sample within this range helps recovering high amounts of the active hTERT protein at the end of the purification process. Any basic buffer may be used to achieve this pH increase. Buffers usually utilized in protein extraction methods include for example Tris 1M pH8.0 buffer, HEPES, Phosphate or MOPS buffers.

The cMBP-hTERT fusion protein can be easily purified by affinity purification. Preferably, the buffers used in this step have a pH comprised between 6.0 and 7.5, more preferably a pH comprised between 6.3 and 7.0 (Fig 1K).

Rnase A may optionally be added at this step to decrease the level of contaminating RNA (e.g., 10 µg of bovine pancreatic Rnase A from Fermentas, per ml of extract). Affinity purification involves the separation of the cMBP-hTERT proteins contained in the crude protein extract based on differences in binding interaction with a ligand that is immobilized to a solid support. Said ligand is covalently linked to the solid support, but non-covalently bound to the fusion protein. The fusion protein may therefore be washed out of the solid support in specific elution conditions well-known in the art.

Preferably, the ligand which is covalently bound to the solid support in the present invention is amylose, which can be non-covalently bound to MBP and cMBP. Amylose is a linear polymer containing D-glucose units. It is commonly used to purify MBP-tagged fusion protein (pMAL™ Protein Fusion & Purification System. New England Biolabs).

The solid support(s) used in the purification step of the method of the invention is (are) any material to which amylose can be covalently attached. Useful solid support(s) is (are) those having a high surface-area to volume ratio, chemical groups that are easily modified for covalent attachment of ligands, minimal nonspecific binding properties, good flow characteristics and mechanical and chemical stability. In a preferred embodiment, the solid support(s) used in the method of the invention is (are) selected from the group consisting of: affinity matrices and beads. Typically, they are made of agarose, sepharose, cellulose, dextran, polyacrylamide, latex and glass. Porous supports (such as sugar- or acrylamide-based polymer resins or gels) and magnetic supports (e.g., magnetic beads) may also be used.

In a preferred embodiment, the purification step d) of the method of the invention uses amylose-coupled agarose beads. Amylose-coupled agarose beads are commercialized for example by New England Biolabs (NEB).

The crude protein extract is then contacted with the ligand-coupled solid support so that the cMBP-hTERT proteins which are present in said extract become non-covalently bound to the solid support. The skilled person knows well the experimental conditions favoring this binding. Typically, contact may last 30 minutes, preferably one hour. This step is preferably performed at cold temperature.

Un-bound components are then advantageously removed by washing the solid support with appropriate washing buffers. Advantageously, the pH of these washing buffers is comprised between 6.0 and 7.5, and is more preferably comprised between 6.3 and 7.0. Thus, in a preferred embodiment, the step d) of the method of the invention includes washing the solid support with buffers having a pH comprised between 6.0 and 7.5, preferably comprised between 6.3 and 7.0. The inventors indeed observed that maintaining the pH of these buffers in this range helps recovering high amounts of the active hTERT protein at the end of the purification process.

The washing buffers may contain high level of salt(s) so as to prevent nonspecific (e.g., ionic) binding interactions. High salt buffers contain typically KCI, MgCl₂, NaCl and/or sodium phosphate, more precisely between 400mM and 800mM of NaCl and between 5 mM and 20 mM of sodium phosphate. A preferred high salt buffer contains 600mM NaCl and 10mM monosodium phosphate.

In a preferred embodiment, the solid support is first washed with a high-salt buffer as defined above, and then washed with a salt-free buffer. These conventional steps are required to efficiently remove all the contaminant molecules unspecifically bound to the solid support.

Salt-free buffers include for example Tris, pH 7.0, 10mM; Hepes, e.g., at 10mM; MOPS buffers; etc.

The fusion protein of the invention is further eluted by adding maltose to the system. This enables to elute the cMBP-hTERT fusion protein off the solid support so as to recover the fusion protein. As a matter of fact, the maltose protein may compete with amylose and favor the release of the fusion protein.

Conventional elution buffer containing maltose may be used. In a preferred embodiment, said elution buffer contains salts such as NaCl, KCI and/or MgCl₂- It may also include between 5mM and 30mM of Hepes. In a more preferred embodiment, said elution buffer contains between 100mM and 200mM of KCI, between 1mM and 5mM of MgCl2, between 5mM and 30mM of Hepes, between 40mM and 80mM of maltose, and has a pH comprised between 6.5 and 7.5. In an even more preferred embodiment, said elution buffer contains 130mM of KCI, 2mM of MgCl2, 10mM of Hepes, 50mM of maltose, and has a pH of about 7.0.

The eluted fraction typically contains around 300 ng/µL of active and soluble cMBP-hTERT protein. The method of the invention is the first that enables to obtain reproducibly and at low costs at least 100 µg/L of the substantially pure hTERT enzyme.

In a preferred embodiment, hTERT is cleaved off the MBP tag by adding a protease to the eluted fraction. Obviously, the said protease can only be active against said fusion protein is said fusion protein comprises a cleavage site recognized by said protease located between the two moieties of the fusion protein. For example, if a sequence encoding the TEV protease cleavage site has been added between the nucleotide sequences encoding the hTERT protein and the cMBP tag, then the separation of the two polypeptides will be advantageously obtained by adding the TEV protease in the eluted fraction. The use of these proteases has been previously described and commercial kits are available (Invitrogen).

The inventors have shown that, by applying the above-described expression and purification method, the purification yield is of about 50%. Consequently, the hTERT protein is the major protein present in the eluted fraction. In other words, it means that the eluted fraction contains only 50% of molecules other than hTERT.

Importantly, the fusion protein which is recovered by means of the method of the invention and the hTERT protein which is recovered after the cleaving of the cMBP tag, are active, i.e., they display a significant telomerase activity, and, more precisely, a significant Reverse Transcriptase (RT) activity in the presence of the telomerase RNA known as "hTR". In a preferred embodiment, they display the same Reverse Transcriptase (RT) activity as the natural hTERT enzyme of SEQ ID NO:1 does in the presence of hTR.

Activity of the proteins of the invention can be assessed by any conventional assays. Typically, these assays are carried out in the presence of hTR. This hTR RNA has for example the SEQ ID NO: 4 (GenBank: U86046.1) or homologous sequences thereof in other mammalian specie (except mouse). Some of these assays are briefly described hereafter.

First, a PCR based telomeric repeat amplification protocol (TRAP assay) can be used (Kim et al. NAR 1997). The TRAP assay may include preparation of the tested hTERT protein, and the addition of *in vitro* transcribed hTR, of primers and dNTPs (see the examples below). If the hTERT protein or variant thereof is enzymatically active, it will elongate the added primer, and the reaction product (templates) will be amplified by PCR. This technique is highly sensitive but can provide only qualitative evaluation. For quantitative analysis, the area or intensity of 6bp ladders appearing in an X-ray film can be measured by densitometry with a computer program. Commercial kits give increased sensitivity with decreased sample processing time, allowing improved detection of telomerase activity in a large number of samples. Another quantitative method for measuring the enzymatic Reverse Transcriptase activity of hTERT protein(s) and / or variant(s) thereof is the primer elongation assay. This assay measures the amount of radioactive nucleotides incorporated into polynucleotides synthesized on a primer sequence. The amount incorporated is measured as a function of the intensity of a band on a phosphorimager screen exposed to a gel on which the radioactive products are separated. A test experiment and a control experiment can be compared by eye on phosphorimager screens. This assay is based on an assay described by Morin, G. B., Cell, 1989. Another assay for assessing Reverse Transcriptase activity of hTERT protein(s) and variant(s) is the dot blot assay. The dot blot assay is useful for routine screening because it has high throughput and hundreds of assays can be carried out in a single day, mostly automatically. Results are available by the afternoon of the second day. Finally, several sensitive direct telomerase activity assays have been proposed (Cohen SB. et al, Nat. Methods, 2008, Houdini™ of Capital Biosciences).

Although not required, a further step of purification may be achieved, for example gel filtration, glycerol gradient filtration, or ultrafiltration.

### Therapeutic use of the hTERT protein of the invention

The hTERT protein obtained by the method of the invention can be used in several applications.

In a second aspect, the recombinant hTERT protein thus purified can be used to create or elevate telomerase activity in a cell to enhance its proliferative capacity. For example, expression of hTERT protein in dermal fibroblasts, thereby increasing telomere length will result in increased fibroblast proliferative capacity; such expression can slow or reverse the age-dependent slowing of wound closure (see, e.g., West, Arch. Derm. 1994).

Thus, in this aspect, the present invention provides reagents and methods useful for treating diseases and conditions characterized by the absence of human telomerase activity in a cell. These diseases include, as described more fully below, diseases associated with cell senescence (particularly diseases of aging) and infertility, among others.

In one aspect, the present invention therefore relates to a pharmaceutically acceptable composition containing the hTERT protein obtained by means of the method of the invention, optionally in combination with a stabilizing compound, a diluent, a carrier, or another active ingredient or agent.

Any suitable pharmaceutically acceptable carrier can be used in the composition of the present invention, and such carriers are well known in the art. The choice of carrier will be determined, in part, by the particular site to which the composition is to be administered and the particular method used to administer the composition. Formulations suitable for injection include aqueous and non-aqueous solutions, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampoules and vials, and can be stored in a freeze-dried (lyophilized) condition requiring only the addition of the sterile liquid carrier, for example, water, immediately prior to use. Extemporaneous injection solutions and suspensions can be prepared from sterile powders, granules, and tablets of the kind previously described.

In particular, the present invention relates to the pharmaceutical composition containing the hTERT protein obtained by means of the method of the invention, for use for treating diseases and conditions characterized by the absence of human telomerase activity, such as diseases associated with cell senescence (particularly diseases of aging) and infertility.

Also, the present invention relates to the use of the hTERT protein obtained by means of the method of the invention for preparing a pharmaceutical composition that is intended to treat diseases and conditions characterized by the absence of human telomerase activity, such as diseases associated with cell senescence (particularly diseases of aging) and infertility.

Certain diseases of aging are characterized by cell senescence-associated changes due to reduced telomere length (compared to younger cells), resulting from the absence (or much lower levels) of telomerase activity in the cell, leading to decreased telomere length and decreased replicative capacity. Conditions associated with cell senescence includes Alzheimer's disease, Parkinson's disease, Huntington's disease, and stroke; age-related diseases of the integument such as dermal atrophy, elastolysis and skin wrinkling, sebaceous gland hyperplasia, senile lentigo, graying of hair and hair loss, chronic skin ulcers, and age-related impairment of wound healing; degenerative joint disease; osteoporosis; age-related immune system impairment (e.g., involving cells such as B and T lymphocytes, monocytes, neutrophils, eosinophils. basophils, NK cells and their respective progenitors); age-related diseases of the vascular system including atherosclerosis, calcification, thrombosis, and aneurysms; diabetes, muscle atrophy, respiratory diseases, diseases of the liver and Gl tract, metabolic diseases, endocrine diseases (e.g., disorders of the pituitary and adrenal gland), reproductive diseases, and age-related macular degeneration.

The present invention also provides methods and composition useful for treating infertility. Human germline cells (e.g., spermatogonia cells, their progenitors or descendants) are capable of indefinite proliferation and characterized by high telomerase activity. Abnormal or diminished levels of the hTERT protein can result, for example, in inadequate or abnormal production of spermatozoa, leading to infertility or disorders of reproduction. Accordingly, "telomerase-based" infertility can be treated using the methods and compositions described herein to increase telomerase levels. The methods and reagents of the invention are also useful for increasing telomerase activity and proliferative potential in stem cells that express a low level of telomerase or no telomerase, prior to therapeutic intervention.

These diseases and conditions can be treated by increasing the levels of the hTERT protein in the cell to increase telomere length, thereby restoring or imparting greater replicative capacity to the cell. Such methods can be carried out on cells cultured ex *vivo* or cells *in vivo.* In one embodiment, the cells are contacted with the hTERT protein *ex vivo* so as to activate telomerase and lengthen telomeres, then said cells are administered to a subject in need thereof. The catalytically active hTERT polypeptide can be introduced into a cell or tissue, e.g., by microinjection or other means known in the art.

### Vaccination use of the hTERT protein of the invention

In another aspect, the hTERT protein can be used to elicit an anti-hTERT immune response in a patient (i.e., act as a vaccine). Once immunized, the individual or animal will elicit an increased immune response against cells expressing high levels of telomerase (e.g., malignant cells).

In another aspect, the present invention therefore relates to a vaccine composition for use for eliciting an anti-hTERT immune response in a subject, said subject suffering for example from cancer.

Also, the present invention relates to the use of the hTERT protein obtained by means of the method of the invention for preparing an immunogenic vaccine that is intended to elicite an anti-hTERT immune response in a subject. In particular, immunogenic vaccine is intended to be administered to patients suffering from cancer.

### Screening of telomerase inhibitors by means of the hTERT protein of the invention

Alternatively, the hTERT protein obtained by the method of the invention can be used for screening for therapeutic compounds in any of a variety of drug screening techniques. The hTERT protein employed in such a test may be free in solution, affixed to a solid support, borne on a cell surface, or located intracellularly. The formation of binding complexes, between the hTERT protein and the agent being tested, may be detected by EMSA, gel filtration, or glycerol-gradient.

In particular embodiments, the screening method of the invention enables to isolate modulators which, *inter alia,* i) bind to the enzyme active site, ii) inhibit the association of hTERT with its RNA moiety, with telomerase-associated proteins (e.g., p80 or p95), with nucleotides, or with telomeric DNA, iii) promote the disassociation of the enzyme complex, iv) interfere with transcription of the telomerase RNA moiety (e.g., hTR) or v) affect the processivity of the enzyme.

In one embodiment, the present invention relates to *in vitro* assays for identifying antagonists of the telomerase complex. These antagonists are for example peptides (such as structural mimetics), polypeptides (such as dominant negative mutants of hTERT or telomerase-associated proteins), small chemical molecules (natural or synthetic), oligonucleotides (such as DNA or RNA oligonucleotides (e.g., aptamers) that bind hTERT or hTR), or antibodies.

These assays comprise the steps of contacting the hTERT protein obtained by the method of the invention with a test compound in a sample, and determining whether the test compound affects the activity of the telomerase in the sample. Usually, this determination comprises comparing the telomerase activity in the sample to the telomerase activity of a sample that does not contain the test compound.

The method for identifying efficient telomerase inhibitors comprises the steps of:
- Contacting, in a sample, the hTERT protein obtained by means of the method of the invention, the RNA of SEQ ID NO:4 (hTR), dNTPs, and the candidate inhibitor,
- testing the telomerase activity of the sample.

Preferably, the method for identifying efficient telomerase inhibitors, which are telomerase assembly inhibitors, comprises, in the following order, the steps of:
a) Contacting, in a sample, the hTERT protein obtained by means of the method of the invention, and the candidate inhibitor,
b) Adding the RNA of SEQ ID NO:4 (hTR), dNTPs,
c) testing the telomerase activity of the sample.

In another preferred embodiment, the method for identifying efficient telomerase inhibitors, which are telomerase catalytic inhibitors, comprises, in the following order, the steps of:
a) Contacting, in a sample, the hTERT protein obtained by means of the method of the invention, and the RNA of SEQ ID NO:4 (hTR),
b) Adding the candidate inhibitor and dNTPs,
c) Testing the telomerase activity of the sample.

Preferably, the candidate inhibitor will be selected if the telomerase activity in the sample containing the candidate inhibitor is low or absent.

In a preferred embodiment, the said screening method requires the comparison between the telomerase activity in the absence and in the presence of the candidate inhibitor. In this case, the candidate inhibitor will be selected if the telomerase activity measured in the sample in the presence of the candidate inhibitor is decreased by at least 50%, more preferably by at least 70% and even more preferably by at least 90% as compared with the telomerase activity measured in the sample in the absence of said inhibitor.

In another preferred embodiment, the said screening method includes the evaluation of the effect of candidate inhibitor on the PCR step of the telomerase activity assay. In this case, the candidate inhibitor will be selected if the telomerase activity is affected when the candidate inhibitor is added before telomere elongation, but is not affected when the candidate inhibitor is added after telomere elongation.

Experimental conditions for assaying the telomerase activity of a sample have been previously described. The classical assays used for measuring the activity of the telomerase complex are for example the TRAP assay, the dot blot assay, or direct telomerase activity assays, which have been described above. Precise protocols are detailed in the examples below.

Preferably, the said inhibitor is identified by monitoring a change in the telomerase activity of a ribonucleoprotein complex (RNP) comprising the hTERT protein obtained by the method of the invention and a template RNA (e.g., the hTR of SEQ ID NO:4), said RNP being reconstituted *in vitro.*

For performing the different steps of the method of the present invention, there may be employed conventional molecular biology, microbiology and recombinant DNA techniques within the skill of the art. Such techniques are explained fully in the literature. See, for example, Sambrook, Fitsch & Maniatis, Molecular Cloning: A Laboratory Manual, Second Edition (1989) Cold Spring Harbor Laboratory Press, Cold Spring Harbor, N.Y. (referred to herein as "Sambrook et al., 1989"); DNA Cloning: A Practical Approach, Volumes I and II (D. N. Glover ed. 1985); Oligonucleotide Synthesis (M. J. Gait ed. 1984); Nucleic Acid Hybridization(B. D. Hames & S. J. Higgins, eds. 1984); Animal Cell Culture (R. I. Freshney, ed. 1986); Immobilized

Cells and Enzymes (IRL Press, 1986); B. E. Perbal, A Practical Guide to Molecular Cloning (1984); F. M. Ausubel et al. (eds.), Current Protocols in Molecular Biology, John Wiley & Sons, Inc. (1994).

### EXAMPLES

### 1. Materials and experimental settings

### 1.1. Plasmid constructions

A hTERT cDNA (kind gift of Robert Weinberg, Whitehead Institute of Biomedical Research) and pMAL p-2 (New England Biolabs) were used as templates for PCR amplification of MBP (SEQ IDNO:9) and hTERT (SEQ ID NO:3) with the following primers:
**MBP-F** (SEQ ID NO:19): ATGCAATTCGAAGGTACCAAGCTTGCCACCATGAAAATCGAAGAAGGTAAAC
**MBP-R** (SEQ ID NO:20): p-TCGTTGGATCGTAATCGTTGTTGTTATTGTTATTG
**hTERT-F** (SEQ ID NO:21): p-CCGAAAACTTATATTTTCAGGGTATGCCGCGCGCTCCCCGCTGCCG and
**hTERT R** (SEQ ID NO:22): CTTCAAGACCATCCTGGACTGAGTCGAGCCGCGGCGGCCGCATGCAA.

Then, PCR fragments were column purified. The MBP DNA was digested with BstBI and the hTERT DNA was digested by Xbal. Both fragments were column purified again, and double-ligated into BstBI/XbaI sites of PGAPZα vector.

### 1.2. Expression and purification of cMBP-hTERT according to the method of the invention

The plasmid was checked by sequencing, then 20µg was linearized with Avril, purified and electroporated into the X-33 strain of *Pichia pastoris* (Invitrogen) using a Bio-Rad Gene Pulser (1500 V, 25µF, 200 Ω). Multi-integrant were selected on agar plates (0.2% Yeast Nitrogen Base with ammonium sulfate, 1% yeast extract, 2% peptone, 2% dextrose, 1M Sorbitol, pH 7.0, 300µg/ml Zeocin) and incubated at 27°C for 2-3 days. Colonies were restreacked, then grown in LBG (Luria Broth and glucose 20g/L each) to check for hTERT expression by western blot (hTERT antibody from Epitomics). A validated clone was amplified in 200 ml (1% yeast extract, pH 7.0, 1% dextrose, 500µg/ml zeocin) at 160 RPM, 27°C, then aliquoted in 2 ml tubes and stored at -80°C with 10% glycerol.

For each culture, one vial was unfrozen, yeast precultured 1-2 days on solid medium, and amplified in 250-500mL of medium in a 2L shake-flask, in 2% Yeast Extract, 4% Glucose, 100mM, NaHPO4 pH7.5, media until OD₆₀₀=12-15 so that the intracellular pH is not higher than 6.3.

All purification steps were performed in cold room with cold solutions and refrigerated instruments.

Yeast were pelleted at 1500 RPM for 10 minutes, washed in water, then resuspended in 15 ml of water and vortexed for 10 minutes with 5 ml of glass beads. It is not necessary to add any salt nor any detergent in water. The pH of this lysate is of about 5.9 - 6.4.

The supernatant was centrifugated at 3000g for 10 minutes, and again at 3000g for 15 minutes in a new tube. The pH was checked to be around 6,3 and the supernatant was applied to 2 ml of pre-rinced amylose-agarose beads (NEB). After one hour on a rotating wheel, amylose-beads were washed once with high-salt buffer (600mM NaCl, 10 mM monosodium phosphate pH 7.0), and once with salt-free buffer (10 mM Hepes pH 7.0). cMBP-hTERT is eluted with 500µL elution buffer (130mM KCI, 2 mM MgCl2, 10 mM Hepes pH 7.0, 50mM Maltose). cMBP-hTERT protein concentration was estimated on gel by Coomassie Brilliant Blue staining against a BSA ladder (figure 2A). cMBP-hTERT was typically found to be around 0,1-0,3 mg/mL. cMBP-hTERT was kept for 2 days at 4°C.

### 1.3. Control of the purification process

The purification of the cMBP-hTERT protein using amylose-sepharose has been followed by Western Blot and Coomassie Brilliant Blue (figure 2A). The shift in size between lane 2 (before cleavage of the MBP tag with the TEV protease) and lane 3 (after cleavage of the MBP tag with the TEV protease) confirmed that the purified protein was cMBP-hTERT (a TEV site has been included between MBP and hTERT).

The purity of the cMBP-hTERT cleaved by the TEV protease for one hour at room temperature and then removed by nickel-column rebinding allowed to evaluate more accurately the purity level and the efficiency of the cleavage by TEV protease (figure 2B).

Purified MBP-hTERT protein was digested using trypsin and analyzed by MS/MS. hTERT was the protein with the highest sequence coverage (75%, not shown).

Telomerase activity has been reconstituted by adding 500ng of *in vitro* transcribed hTR to 100ng of purified MBP-hTERT. This activity has been revealed by several methods, such as Telomerase Direct Assay (figure 3A), telomeric repeat amplification protocol (TRAP or qTRAP, figure 3B and 3C). Telomerase activity increases in the first minutes before reaching a stable plateau (figure 3D). Finally, EMSA can also show binding of recombinant hTERT to ³²P labeled hTR.

### 1.4. Telom erase Direct Assay

Direct Assay was performed as described (D'Ambrosio et al., 2012) with slight modifications. Reconstituted telomerase is incubated for 45 min at 30 °C in 20 µl of reaction buffer containing 40 mM Tris-HCl pH 7.9, 1 mM MgCl2, 1 mM Dithiothreitol, 2 mM spermidine, 40 µM dATP, 80 µM dTTP, 2 µM dGTP, 20 µCi of [α-³²P]-dGTP (3,000 Ci/mmol) and 33 nM of a 5'-biotinylated primer, as telomerase substrate. Reactions were stopped by adding EDTA to 25 mM. Unincorporated nucleotides are removed by binding the biotinylated primer to 20 µl of streptavidin-agarose beads (GE Healthcare) for 10 min at room temperature. Beads are washed twice with 10 mM Tris-HCl pH 8.0, 1 mM EDTA, 1 M NaCl, and once with 10 mM Tris-HCl pH 8.0, 1 mM EDTA. The primer is eluted by heating at 95°C for 10 minutes in 90% formamide, 10 mM EDTA, 0.5 mM Biotin (Sigma) and separated on 15% polyacrylamide-urea sequencing gels (19:1 acrylamide:bisacrylamide ratio). Gel is covered with a plastic-film, exposed to a phosphorimager screen, and scanned using the STORM 860 (Molecular Dynamics).

### 1.5. Binding of recombinant hTERT to ³²P labeled h TR

hTR or truncations were synthetised with the addition of 50 µCi of [α-³²P]-CTP, and assembled with MBP-hTERT for one hour. Full length hTR was migrated at 110V for 2 hours on a 1.2 % refrigerated agarose gel in 1x TBE. Truncations were run on a 1.8% agarose gel. Gel were fixed for one hour in 10% *acetic acid* 10% ethanol, dried and exposed to a phosphorimager screen. STORM 860 (Molecular Dynamics) was used to perform the scan, and Imagequant software to quantify. To normalize the variations due to gel loading, the percent of binding was calculated following the formula: upper signal/total signal of the lane.

### 2. Identification of optimized conditions for hTERT expression

### 2.1. Removal of the N-terminal periplasmic targeting signal allows better expression in yeast

The MBP contains an N-terminal periplasmic targeting signal. The present inventors have shown that removing this sequence from MBP-hTERT allows better expression in yeast so that it is possible to detect the purified protein cMBP-hTERT on Coomassie Brilliant Blue (see figure 2).

This advantageously avoids the occurrence of unwanted glycosylations.

### 2.2. hTERT cannot be secreted from yeast cells

GST-hTERT (155kDa) is expressed intracellulary using PGAPZ vector (see figure 1A and B).

However, when GST-hTERT was fused to the secretion signal of *Saccharomyces cerevisiae* alpha factor (α-hTERT, 136 kDa), α-hTERT remained intracellular (figure 1D and E). Comparing intracellular and extracellular levels of hTERT and α-hTERT showed that adding a secretion signal does not improve the expression level of hTERT. Figure 1F also shows that α-MBP-hTERT is barely detectable by western blot anti-HA, either intracellularly, or extracellularly.

Thus, contrary to the situation described in insect cells (Wu et al., Protein Expr. Purif. 2007), in the present yeast system, the addition of a peptide signal (from *Saccharomyces cerevisiae* α factor) was not required to express hTERT soluble (figure 1E).

Moreover, it was demonstrated that the removal of the periplasmic signal of MBP improved the expression level of MBP-hTERT in yeast (Figure 2).

### 2.3. Only MBP allows efficient purification of hTERT

The present inventors have demonstrated that the use of several other TAG (His, His-MBP, GST,) do not support efficient purification (see for example for GST, figure 1C). His-Tagged hTERT could not be detected by Coomassie Brillant Blue after purification, and generated only 2-5% of the telomerase activity level obtained with purified MBP-hTERT. No protein expression could be detected by Western blot in yeast transformed to express hTERT fused with Strep-Tag II at its N-terminal (two different constructs tested), therefore this tag may be detrimental to protein expression or stability.

### 2.4. Importance of the expression system (constitutive promoter and rich media)

The full protein could not be expressed efficiently with the classical *Pichia* system based on the AOX1 promoter (Figure 1G). With the GAPDH promoter, hTERT was found to be efficiently expressed only in rich media (containing yeast extract). Therefore a constitutive promoter, or possibly an inducible one which is not repressed in rich media, is preferred.

### 2.5. Importance of the growth conditions

Yeast recently unfrozen or stored at 4°C produces hTERT proteins of less good quality. Best results were obtained with exponentially growing yeast coming from a fresh preculture.

Yeast must be grown up until when the intracellular pH will be around 6.0. In shake flask conditions, this usually corresponds to an OD₆₀₀ₙₘ = 10-12. Yeast growth in acidic conditions leads to the recovery of degraded MBP-hTERT protein (Figure 1I).

### 2.6. Importance of the extraction conditions

Unexpectedly for a nuclear/basic protein, high salt concentrations (NaCl or KCI) were found to decrease extraction efficiency. Buffered solutions (hepes or tris pH 7-8) also reduced extraction.

Interestingly, best results were found using pure water, which gets the intracellular pH of yeast after cell disruption (around 6.0). Addition of glycerol, detergents, EDTA, EGTA, DTT, protease inhibitors during the extraction/purification as well as addition of salts (KCL, Mg2+, NaCL, ammonium bicarbonate....) after the extraction had all little effect on the yield, purity and activity level of the purified protein. (Figure 1J).

### 2.7. Importance of the purification conditions

Best binding to the amylose resin was observed at slightly acid pH (6,3 - 7.0) (Fig 1K). Efficient purification therefore requires that the binding and all washing solutions are set to a pH comprised between 6.0 and 7.5.

### 3. Use of MBP-purified hTERT in high-throughput screening

The MBP-purified hTERT has been used to identify potential telomerase inhibitors with a chemical library of 8000 components.

Determination of the mode of inhibition: Each molecule is tested in three conditions. It is added either before telomerase assembly (PRE), or after telomerase assembly (POST), or after telomere elongation (PCR-C.). Telomerase assembly inhibitors are identified by inhibiting only when introduced at step 1. Telomerase catalytic inhibitors work when introduced either at step 1 or 2. PCR inhibitors (false positives) inhibit only at step 3.

### BIBLIOGRAPHIC REFERENCES

Alves D. et al, Nat. Chem. Biol. 2008 ; 4(5):287-9
Armbruster et al, Mol. Cell. Biol. 2001; vol.22 n°17 ; p.6234-6246
Autexier C. et al, Annu. Rev. Biochem. 75 (2006) 493-517
Bachand F. et al, JBC 1999 ; 274(53) :38027-31
Banik SSR et al, Mol. Cell. Biol. 2002; vol.21 No 22, p.7775-7786
Cohen SB. et al, Nat. Methods, 2008; 5(4):355-60
D'Ambrosio et al. Biochimie, 2012; 94:854-863
Holt S.E. et al., Genes Dev. 13 (1999) 817-826
Kim et al. NAR 1997; 25(13):2595-2597
Loiselle FB and Casey JR, Methods Mol. Biol. 2010; 637:311-31
Masutomi K. et al. J. Biol. Chem. 2000; 275(29):22568-73
Mikuni O. et al. Biochem. Biophys. Res. Commun. 2002 ; 298(1) :144-50
Mitchell J.R. et al. Nature 402 (1999) 551-555
Morin, G. B., Cell 59:521-529, 1989
Nakayama J. et al., Cell 88(1997) 875-884
Needleman and Wunsch. J.Mol.Biol. 1970, 48(3):443-53.
Poole JC et al, Gene 2001; 269(1-2):1-12
Weinrich SL. et al, Nat. Genet. 1997; 17(4):498-502
Wenz C. et al. EMBO 2001 ; 20(13) :3526-34
West, 1994, Arch. Derm. 130:87
Wu C.K. et al, Protein Expr. Purif. 2007 ; 56(1) :8-19
Zhang X. et al, Genes Dev. 1999, 13(18):2388-99

## Claims

1. A method to produce active and soluble human telomerase reverse transcriptase (hTERT) protein, comprising the steps of:
a) Growing yeast cells comprising a nucleotide vector, said vector containing a constitutive promoter operatively linked to a nucleotide sequence encoding a fusion protein containing:
- a maltose-binding protein deleted of its N-terminal periplasmic targeting signal (cMBP), and
- a human telomerase reverse transcriptase (hTERT) protein,
b) Preparing a crude protein extract of the cells of step a),
c) Adjusting, if necessary, the pH of said extract to a pH comprised between 6.0 and 7.5,
d) Purifying the fusion protein cMBP-hTERT by means of an amylose-coupled solid support.

2. The method of claim 1, wherein step b), is performed before intracellular pH of said yeast cells reaches a value below 5.8.

3. The method of claim 1, wherein said yeast cells are *Pichia pastoris* cells.

4. The method of any one of claim 1 to 3, wherein said constitutive promoter is the GAPDH promoter, preferably of SEQ ID NO:10.

5. The method of any one of claim 1 to 4, wherein said maltose-binding protein tag has the polypeptide sequence SEQ ID NO:8.

6. The method of any one of claim 1 to 5, wherein said nucleotide sequence further contains a nucleotide sequence encoding a protease cleavage site between the sequence encoding the maltose-binding protein tag and the sequence encoding the hTERT protein.

7. The method of any one of claim 1 to 6, wherein step b) includes the lysis of the yeast cells in a water-based solution, preferably salt and detergent-free water.

8. The method of claim 7, wherein said water-based solution does not contain any protease inhibitor.

9. The method of any one of claim 1 to 8, wherein said solid support consists in amylose-coupled agarose beads.

10. The method of any one of claim 1 to 9, wherein said solid support is washed with washing buffers having a pH comprised between 6.0 and 7.5.

11. The method of any one of claims 1 to 10, further comprising the step of cleaving the cMBP-tag from the hTERT protein by adding a protease in the purified fraction.

12. The method of any one of claim 1 to 11, wherein said vector is an integrative vector.

13. A pharmaceutical composition containing the hTERT protein obtained by the method of any one of claims 1 to 12 for use for treating cell senescence or infertility.

14. A vaccine composition containing the hTERT protein obtained by the method of any one of claims 1 to 12 for use for eliciting an anti-hTERT immune response in a subject.

15. An *in vitro* method for identifying efficient telomerase inhibitors using the hTERT protein obtained by the method of any one of claims 1 to 12.


SEQUENCE LISTING

<110>  CENTRE NATIONAL DE LA RECHERCHE SCIENTIFIQUE (CNRS)
       UNIVERSITE PARIS DESCARTES
       INSTITUT NATIONAL DE LA SANTE ET DE LA RECHERCHE MEDICALE
       (INSERM)

<120>  NEW METHODS TO PRODUCE ACTIVE HTERT

<130>  70708 D32538

<160>  22

<170>  PatentIn version 3.5

<210>  1
<211>  1132
<212>  PRT
<213>  homo sapiens


<220>
<221>  MISC_FEATURE
<223>  hTERT - NP_937983.2

<400>  1

<210>  2
<211>  4018
<212>  DNA
<213>  homo sapiens

<220>
<221>  misc_feature
<223>  hTERT mRNA - NM_198253.2

<400>  2
caggcagcgc tgcgtcctgc tgcgcacgtg ggaagccctg gccccggcca cccccgcgat      60

gccgcgcgct ccccgctgcc gagccgtgcg ctccctgctg cgcagccact accgcgaggt     120

gctgccgctg gccacgttcg tgcggcgcct ggggccccag ggctggcggc tggtgcagcg     180

cggggacccg gcggctttcc gcgcgctggt ggcccagtgc ctggtgtgcg tgccctggga     240

cgcacggccg ccccccgccg ccccctcctt ccgccaggtg tcctgcctga aggagctggt     300

ggcccgagtg ctgcagaggc tgtgcgagcg cggcgcgaag aacgtgctgg ccttcggctt     360

cgcgctgctg gacggggccc gcgggggccc ccccgaggcc ttcaccacca gcgtgcgcag     420

ctacctgccc aacacggtga ccgacgcact gcgggggagc ggggcgtggg ggctgctgct     480

gcgccgcgtg ggcgacgacg tgctggttca cctgctggca cgctgcgcgc tctttgtgct     540

ggtggctccc agctgcgcct accaggtgtg cgggccgccg ctgtaccagc tcggcgctgc     600

cactcaggcc cggcccccgc cacacgctag tggaccccga aggcgtctgg gatgcgaacg     660

ggcctggaac catagcgtca gggaggccgg ggtccccctg ggcctgccag ccccgggtgc     720

gaggaggcgc gggggcagtg ccagccgaag tctgccgttg cccaagaggc ccaggcgtgg     780

cgctgcccct gagccggagc ggacgcccgt tgggcagggg tcctgggccc acccgggcag     840

gacgcgtgga ccgagtgacc gtggtttctg tgtggtgtca cctgccagac ccgccgaaga     900

agccacctct ttggagggtg cgctctctgg cacgcgccac tcccacccat ccgtgggccg     960

ccagcaccac gcgggccccc catccacatc gcggccacca cgtccctggg acacgccttg    1020

tcccccggtg tacgccgaga ccaagcactt cctctactcc tcaggcgaca aggagcagct    1080

gcggccctcc ttcctactca gctctctgag gcccagcctg actggcgctc ggaggctcgt    1140

ggagaccatc tttctgggtt ccaggccctg gatgccaggg actccccgca ggttgccccg    1200

cctgccccag cgctactggc aaatgcggcc cctgtttctg gagctgcttg ggaaccacgc    1260

gcagtgcccc tacggggtgc tcctcaagac gcactgcccg ctgcgagctg cggtcacccc    1320

agcagccggt gtctgtgccc gggagaagcc ccagggctct gtggcggccc ccgaggagga    1380

ggacacagac ccccgtcgcc tggtgcagct gctccgccag cacagcagcc cctggcaggt    1440

gtacggcttc gtgcgggcct gcctgcgccg gctggtgccc ccaggcctct ggggctccag    1500

gcacaacgaa cgccgcttcc tcaggaacac caagaagttc atctccctgg ggaagcatgc    1560

caagctctcg ctgcaggagc tgacgtggaa gatgagcgtg cgggactgcg cttggctgcg 1620

caggagccca ggggttggct gtgttccggc cgcagagcac cgtctgcgtg aggagatcct 1680

ggccaagttc ctgcactggc tgatgagtgt gtacgtcgtc gagctgctca ggtctttctt 1740

ttatgtcacg gagaccacgt ttcaaaagaa caggctcttt ttctaccgga agagtgtctg 1800

gagcaagttg caaagcattg gaatcagaca gcacttgaag agggtgcagc tgcgggagct 1860

gtcggaagca gaggtcaggc agcatcggga agccaggccc gccctgctga cgtccagact 1920

ccgcttcatc cccaagcctg acgggctgcg gccgattgtg aacatggact acgtcgtggg 1980

agccagaacg ttccgcagag aaaagagggc cgagcgtctc acctcgaggg tgaaggcact 2040

gttcagcgtg ctcaactacg agcgggcgcg gcgccccggc ctcctgggcg cctctgtgct 2100

gggcctggac gatatccaca gggcctggcg caccttcgtg ctgcgtgtgc gggcccagga 2160

cccgccgcct gagctgtact ttgtcaaggt ggatgtgacg ggcgcgtacg acaccatccc 2220

ccaggacagg ctcacggagg tcatcgccag catcatcaaa ccccagaaca cgtactgcgt 2280

gcgtcggtat gccgtggtcc agaaggccgc ccatgggcac gtccgcaagg ccttcaagag 2340

ccacgtctct accttgacag acctccagcc gtacatgcga cagttcgtgg ctcacctgca 2400

ggagaccagc ccgctgaggg atgccgtcgt catcgagcag agctcctccc tgaatgaggc 2460

cagcagtggc ctcttcgacg tcttcctacg cttcatgtgc caccacgccg tgcgcatcag 2520

gggcaagtcc tacgtccagt gccaggggat cccgcagggc tccatcctct ccacgctgct 2580

ctgcagcctg tgctacggcg acatggagaa caagctgttt gcggggattc ggcgggacgg 2640

gctgctcctg cgtttggtgg atgatttctt gttggtgaca cctcacctca cccacgcgaa 2700

aaccttcctc aggaccctgg tccgaggtgt ccctgagtat ggctgcgtgg tgaacttgcg 2760

gaagacagtg gtgaacttcc ctgtagaaga cgaggccctg ggtggcacgg cttttgttca 2820

gatgccggcc cacggcctat tcccctggtg cggcctgctg ctggataccc ggaccctgga 2880

ggtgcagagc gactactcca gctatgcccg gacctccatc agagccagtc tcaccttcaa 2940

ccgcggcttc aaggctggga ggaacatgcg tcgcaaactc tttggggtct tgcggctgaa 3000

gtgtcacagc ctgtttctgg atttgcaggt gaacagcctc cagacggtgt gcaccaacat 3060

ctacaagatc ctcctgctgc aggcgtacag gtttcacgca tgtgtgctgc agctcccatt 3120

tcatcagcaa gtttggaaga accccacatt tttcctgcgc gtcatctctg acacggcctc 3180

cctctgctac tccatcctga aagccaagaa cgcagggatg tcgctggggg ccaagggcgc 3240

cgccggccct ctgccctccg aggccgtgca gtggctgtgc caccaagcat tcctgctcaa 3300

gctgactcga caccgtgtca cctacgtgcc actcctgggg tcactcagga cagcccagac 3360

gcagctgagt cggaagctcc cggggacgac gctgactgcc ctggaggccg cagccaaccc 3420

ggcactgccc tcagacttca agaccatcct ggactgatgg ccacccgccc acagccaggc 3480

cgagagcaga caccagcagc cctgtcacgc cgggctctac gtcccaggga gggaggggcg 3540

gcccacaccc aggcccgcac cgctgggagt ctgaggcctg agtgagtgtt tggccgaggc 3600

ctgcatgtcc ggctgaaggc tgagtgtccg gctgaggcct gagcgagtgt ccagccaagg 3660

gctgagtgtc cagcacacct gccgtcttca cttccccaca ggctggcgct cggctccacc 3720

ccagggccag cttttcctca ccaggagccc ggcttccact ccccacatag gaatagtcca 3780

tccccagatt cgccattgtt cacccctcgc cctgccctcc tttgccttcc acccccacca 3840

tccaggtgga gaccctgaga aggaccctgg gagctctggg aatttggagt gaccaaaggt 3900

gtgccctgta cacaggcgag gaccctgcac ctggatgggg gtccctgtgg gtcaaattgg 3960

ggggaggtgc tgtgggagta aaatactgaa tatatgagtt tttcagtttt gaaaaaa 4018

<210> 3
<211> 3399
<212> DNA
<213> homo sapiens

<220>
<221> misc_feature
<223> nucleotide encoding hTERT

<400> 3
atgccgcgcg ctccccgctg ccgagccgtg cgctccctgc tgcgcagcca ctaccgcgag 60

gtgctgccgc tggccacgtt cgtgcggcgc ctggggcccc agggctggcg gctggtgcag 120

cgcggggacc cggcggcttt ccgcgcgctg gtggcccagt gcctggtgtg cgtgccctgg 180

gacgcacggc cgccccccgc cgccccctcc ttccgccagg tgtcctgcct gaaggagctg 240

gtggcccgag tgctgcagag gctgtgcgag cgcggcgcga agaacgtgct ggccttcggc 300

ttcgcgctgc tggacggggc ccgcgggggc ccccccgagg ccttcaccac cagcgtgcgc 360

agctacctgc ccaacacggt gaccgacgca ctgcggggga gcggggcgtg ggggctgctg 420

ctgcgccgcg tgggcgacga cgtgctggtt cacctgctgg cacgctgcgc gctctttgtg 480

ctggtggctc ccagctgcgc ctaccaggtg tgcgggccgc cgctgtacca gctcggcgct 540

gccactcagg cccggccccc gccacacgct agtggacccc gaaggcgtct gggatgcgaa 600

cgggcctgga accatagcgt cagggaggcc ggggtccccc tgggcctgcc agccccgggt 660

gcgaggaggc gcgggggcag tgccagccga agtctgccgt tgcccaagag gcccaggcgt 720

ggcgctgccc ctgagccgga gcggacgccc gttgggcagg ggtcctgggc ccacccgggc 780

aggacgcgtg gaccgagtga ccgtggtttc tgtgtggtgt cacctgccag acccgccgaa 840

gaagccacct ctttggaggg tgcgctctct ggcacgcgcc actcccaccc atccgtgggc 900

cgccagcacc acgcgggccc cccatccaca tcgcggccac cacgtccctg ggacacgcct 960

tgtcccccgg tgtacgccga gaccaagcac ttcctctact cctcaggcga caaggagcag 1020

ctgcggccct ccttcctact cagctctctg aggcccagcc tgactggcgc tcggaggctc 1080

gtggagacca tctttctggg ttccaggccc tggatgccag ggactccccg caggttgccc 1140

cgcctgcccc agcgctactg gcaaatgcgg cccctgtttc tggagctgct tgggaaccac 1200

gcgcagtgcc cctacggggt gctcctcaag acgcactgcc cgctgcgagc tgcggtcacc 1260

ccagcagccg gtgtctgtgc ccgggagaag ccccagggct ctgtggcggc ccccgaggag 1320

gaggacacag acccccgtcg cctggtgcag ctgctccgcc agcacagcag cccctggcag 1380

gtgtacggct tcgtgcgggc ctgcctgcgc cggctggtgc ccccaggcct ctggggctcc 1440

aggcacaacg aacgccgctt cctcaggaac accaagaagt tcatctccct ggggaagcat 1500

gccaagctct cgctgcagga gctgacgtgg aagatgagcg tgcgggactg cgcttggctg 1560

cgcaggagcc caggggttgg ctgtgttccg gccgcagagc accgtctgcg tgaggagatc 1620

ctggccaagt tcctgcactg gctgatgagt gtgtacgtcg tcgagctgct caggtctttc 1680

ttttatgtca cggagaccac gtttcaaaag aacaggctct ttttctaccg gaagagtgtc 1740

tggagcaagt tgcaaagcat tggaatcaga cagcacttga agagggtgca gctgcgggag 1800

ctgtcggaag cagaggtcag gcagcatcgg gaagccaggc ccgccctgct gacgtccaga 1860

ctccgcttca tccccaagcc tgacgggctg cggccgattg tgaacatgga ctacgtcgtg 1920

ggagccagaa cgttccgcag agaaaagagg gccgagcgtc tcacctcgag ggtgaaggca 1980

ctgttcagcg tgctcaacta cgagcgggcg cggcgccccg gcctcctggg cgcctctgtg 2040

ctgggcctgg acgatatcca cagggcctgg cgcaccttcg tgctgcgtgt gcgggcccag 2100

gacccgccgc ctgagctgta ctttgtcaag gtggatgtga cgggcgcgta cgacaccatc 2160

ccccaggaca ggctcacgga ggtcatcgcc agcatcatca aaccccagaa cacgtactgc 2220

gtgcgtcggt atgccgtggt ccagaaggcc gcccatgggc acgtccgcaa ggccttcaag 2280

agccacgtct ctaccttgac agacctccag ccgtacatgc gacagttcgt ggctcacctg 2340

caggagacca gcccgctgag ggatgccgtc gtcatcgagc agagctcctc cctgaatgag 2400

gccagcagtg gcctcttcga cgtcttccta cgcttcatgt gccaccacgc cgtgcgcatc 2460

aggggcaagt cctacgtcca gtgccagggg atcccgcagg gctccatcct ctccacgctg 2520

ctctgcagcc tgtgctacgg cgacatggag aacaagctgt ttgcggggat tcggcgggac 2580

gggctgctcc tgcgtttggt ggatgatttc ttgttggtga cacctcacct cacccacgcg 2640

aaaaccttcc tcaggaccct ggtccgaggt gtccctgagt atggctgcgt ggtgaacttg 2700

cggaagacag tggtgaactt ccctgtagaa gacgaggccc tgggtggcac ggcttttgtt 2760

cagatgccgg cccacggcct attcccctgg tgcggcctgc tgctggatac ccggaccctg 2820

gaggtgcaga gcgactactc cagctatgcc cggacctcca tcagagccag tctcaccttc 2880

aaccgcggct tcaaggctgg gaggaacatg cgtcgcaaac tctttggggt cttgcggctg 2940

aagtgtcaca gcctgtttct ggatttgcag gtgaacagcc tccagacggt gtgcaccaac 3000

atctacaaga tcctcctgct gcaggcgtac aggtttcacg catgtgtgct gcagctccca 3060

tttcatcagc aagtttggaa gaaccccaca tttttcctgc gcgtcatctc tgacacggcc 3120

tccctctgct actccatcct gaaagccaag aacgcaggga tgtcgctggg ggccaagggc 3180

gccgccggcc ctctgccctc cgaggccgtg cagtggctgt gccaccaagc attcctgctc 3240

aagctgactc gacaccgtgt cacctacgtg ccactcctgg ggtcactcag gacagcccag 3300

acgcagctga gtcggaagct cccggggacg acgctgactg ccctggaggc cgcagccaac 3360

ccggcactgc cctcagactt caagaccatc ctggactga 3399

<210> 4
<211> 545
<212> DNA
<213> homo sapiens

<220>
<221> misc_feature
<223> hTR- GenBank: U86046.1

<400> 4
gagtgactct cacgagagcc gcgagagtca gcttggccaa tccgtgcggt cggcggccgc 60

tccctttata agccgactcg cccggcagcg caccgggttg cggagggtgg gcctgggagg 120

ggtggtggcc attttttgtc taaccctaac tgagaagggc gtaggcgccg tgcttttgct 180

ccccgcgcgc tgtttttctc gctgactttc agcgggcgga aaagcctcgg cctgccgcct 240

tccaccgttc attctagagc aaacaaaaaa tgtcagctgc tggcccgttc gcccctcccg 300

gggacctgcg gcgggtcgcc tgcccagccc ccgaaccccg cctggaggcc gcggtcggcc 360

cggggcttct ccggaggcac ccactgccac cgcgaagagt tgggctctgt cagccgcggg 420

tctctcgggg gcgagggcga ggttcaggcc tttcaggccg caggaagagg aacggagcga 480

gtccccgcgc gcggcgcgat tccctgagct gtgggacgtg cacccaggac tcggctcaca 540

catgc 545

<210> 5
<211> 6
<212> DNA
<213> homo sapiens

<220>
<221> misc_feature
<223> DNA repeat of the telomerase

<400> 5

ttaggg                                                                  6

<210>  6
<211>  392
<212>  PRT
<213>  artificial

<220>
<223>  amino acid sequence of MBP with periplasmic signal (MBP)

<400>  6

Met Lys Ile Lys Thr Gly Ala Arg Ile Leu Ala Leu Ser Ala Leu Thr
1               5                   10                  15

Thr Met Met Phe Ser Ala Ser Ala Leu Ala Lys Ile Glu Glu Gly Lys
            20                  25                  30

Leu Val Ile Trp Ile Asn Gly Asp Lys Gly Tyr Asn Gly Leu Ala Glu
        35                  40                  45

Val Gly Lys Lys Phe Glu Lys Asp Thr Gly Ile Lys Val Thr Val Glu
    50                  55                  60

His Pro Asp Lys Leu Glu Glu Lys Phe Pro Gln Val Ala Ala Thr Gly
65                  70                  75                  80

Asp Gly Pro Asp Ile Ile Phe Trp Ala His Asp Arg Phe Gly Gly Tyr
                85                  90                  95

Ala Gln Ser Gly Leu Leu Ala Glu Ile Thr Pro Asp Lys Ala Phe Gln
            100                 105                 110

Asp Lys Leu Tyr Pro Phe Thr Trp Asp Ala Val Arg Tyr Asn Gly Lys
        115                 120                 125

Leu Ile Ala Tyr Pro Ile Ala Val Glu Ala Leu Ser Leu Ile Tyr Asn
    130                 135                 140

Lys Asp Leu Leu Pro Asn Pro Pro Lys Thr Trp Glu Glu Ile Pro Ala
145                 150                 155                 160

Leu Asp Lys Glu Leu Lys Ala Lys Gly Lys Ser Ala Leu Met Phe Asn
                165                 170                 175

Leu Gln Glu Pro Tyr Phe Thr Trp Pro Leu Ile Ala Ala Asp Gly Gly
            180                 185                 190

Tyr Ala Phe Lys Tyr Glu Asn Gly Lys Tyr Asp Ile Lys Asp Val Gly
        195                 200                 205

Val Asp Asn Ala Gly Ala Lys Ala Gly Leu Thr Phe Leu Val Asp Leu
    210                 215                 220

Ile Lys Asn Lys His Met Asn Ala Asp Thr Asp Tyr Ser Ile Ala Glu
225                 230                 235                 240

Ala Ala Phe Asn Lys Gly Glu Thr Ala Met Thr Ile Asn Gly Pro Trp
                245                 250                 255

Ala Trp Ser Asn Ile Asp Thr Ser Lys Val Asn Tyr Gly Val Thr Val
            260                 265                 270

Leu Pro Thr Phe Lys Gly Gln Pro Ser Lys Pro Phe Val Gly Val Leu
        275                 280                 285

Ser Ala Gly Ile Asn Ala Ala Ser Pro Asn Lys Glu Leu Ala Lys Glu
    290                 295                 300

Phe Leu Glu Asn Tyr Leu Leu Thr Asp Glu Gly Leu Glu Ala Val Asn
305                 310                 315                 320

Lys Asp Lys Pro Leu Gly Ala Val Ala Leu Lys Ser Tyr Glu Glu Glu
                325                 330                 335

Leu Ala Lys Asp Pro Arg Ile Ala Ala Thr Met Glu Asn Ala Gln Lys
            340                 345                 350

Gly Glu Ile Met Pro Asn Ile Pro Gln Met Ser Ala Phe Trp Tyr Ala
        355                 360                 365

Val Arg Thr Ala Val Ile Asn Ala Ala Ser Gly Arg Gln Thr Val Asp
    370                 375                 380

Glu Ala Leu Lys Asp Ala Gln Thr
385                 390

<210>  7
<211>  26
<212>  PRT
<213>  artificial

<220>
<223>  amino acid sequence of the periplasmic signal of MBP

<400>  7

Met Lys Ile Lys Thr Gly Ala Arg Ile Leu Ala Leu Ser Ala Leu Thr
1               5                   10                  15

Thr Met Met Phe Ser Ala Ser Ala Leu Ala
            20                  25

<210>  8
<211>  367
<212>  PRT
<213>  artificial

<220>
<223>  amino acid sequence of MBP without periplasmic signal (cMBP)

<400>  8

Met Lys Ile Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285

Glu Gly Leu Glu Ala Val Asn Lys Asp Lys Pro Leu Gly Ala Val Ala
    290                 295                 300

Leu Lys Ser Tyr Glu Glu Glu Leu Ala Lys Asp Pro Arg Ile Ala Ala
305                 310                 315                 320

Thr Met Glu Asn Ala Gln Lys Gly Glu Ile Met Pro Asn Ile Pro Gln
                325                 330                 335

Met Ser Ala Phe Trp Tyr Ala Val Arg Thr Ala Val Ile Asn Ala Ala
            340                 345                 350

Ser Gly Arg Gln Thr Val Asp Glu Ala Leu Lys Asp Ala Gln Thr
        355                 360                 365

<210>  9
<211>  1143
<212>  DNA
<213>  artificial

<220>
<223>  DNA sequence encoding cMBP

<400>  9
atgaaaatcg aagaaggtaa actggtaatc tggattaacg gcgataaagg ctataacggt      60

ctcgctgaag tcggtaagaa attcgagaaa gataccggaa ttaaagtcac cgttgagcat     120

ccggataaac tggaagagaa attcccacag gttgcggcaa ctggcgatgg ccctgacatt     180

atcttctggg cacacgaccg ctttggtggc tacgctcaat ctggcctgtt ggctgaaatc     240

accccggaca aagcgttcca ggacaagctg tatccgttta cctgggatgc cgtacgttac     300

aacggcaagc tgattgctta cccgatcgct gttgaagcgt tatcgctgat ttataacaaa 360

gatctgctgc cgaacccgcc aaaaacctgg gaagagatcc cggcgctgga taaagaactg 420

aaagcgaaag gtaagagcgc gctgatgttc aacctgcaag aaccgtactt cacctggccg 480

ctgattgctg ctgacggggg ttatgcgttc aagtatgaaa acggcaagta cgacattaaa 540

gacgtgggcg tggataacgc tggcgcgaaa gcgggtctga ccttcctggt tgacctgatt 600

aaaaacaaac acatgaatgc agacaccgat tactccatcg cagaagctgc ctttaataaa 660

ggcgaaacag cgatgaccat caacggcccg tgggcatggt ccaacatcga caccagcaaa 720

gtgaattatg gtgtaacggt actgccgacc ttcaagggtc aaccatccaa accgttcgtt 780

ggcgtgctga gcgcaggtat taacgccgcc agtccgaaca aagagctggc aaaagagttc 840

ctcgaaaact atctgctgac tgatgaaggt ctggaagcgg ttaataaaga caaaccgctg 900

ggtgccgtag cgctgaagtc ttacgaggaa gagttggcga aagatccacg tattgccgcc 960

accatggaaa acgcccagaa aggtgaaatc atgccgaaca tcccgcagat gtccgctttc 1020

tggtatgccg tgcgtactgc ggtgatcaac gccgccagcg gtcgtcagac tgtcgatgaa 1080

gccctgaaag acgcgcagac taattcgagc tcgaacaaca acaacaataa caataacaac 1140

aac 1143

<210> 10
<211> 483
<212> DNA
<213> artificial

<220>
<223> nucleotide sequence of the GAPDH promoter

<400> 10
agatcttttt tgtagaaatg tcttggtgtc ctcgtccaat caggtagcca tctctgaaat 60

atctggctcc gttgcaactc cgaacgacct gctggcaacg taaaattctc cggggtaaaa 120

cttaaatgtg gagtaatgga accagaaacg tctcttccct tctctctcct tccaccgccc 180

gttaccgtcc ctaggaaatt ttactctgct ggagagcttc ttctacggcc cccttgcagc 240

aatgctcttc ccagcattac gttgcgggta aaacggaggt cgtgtacccg acctagcagc 300

ccagggatgg aaaagtcccg gccgtcgctg gcaataatag cgggcggacg catgtcatga 360

gattattgga aaccaccaga atcgaatata aaaggcgaac acctttccca attttggttt 420

ctcctgaccc aaagacttta aatttaattt atttgtccct atttcaatca attgaacaac 480

tat 483

<210> 11
<211> 21
<212> DNA
<213> artificial

<220>
<223>  DNA encoding TEV cleavage site used by the inventor

<400>  11
gaaaacttat attttcaggg t                                                21


<210>  12
<211>  27
<212>  PRT
<213>  artificial

<220>
<223>  amino acid sequence of a spacer containing a TEV site

<400>  12

Asn Ser Ser Ser Asn Asn Asn Asn Asn Asn Asn Asn Asn Asn Asp Tyr
1               5                   10                  15


Asp Pro Thr Thr Glu Asn Leu Tyr Phe Gln Gly
            20                  25


<210>  13
<211>  56
<212>  PRT
<213>  artificial

<220>
<223>  amino acid sequence of a spacer containing a HA tag and a TEV
       site

<400>  13

Asn Ser Ser Ser Asn Asn Asn Asn Asn Asn Asn Asn Asn Asn Asp Tyr
1               5                   10                  15


Asp Ile Pro Thr Thr Glu Asn Leu Tyr Phe Gln Gly Ala Tyr Pro Tyr
            20                  25                  30


Asp Val Pro Asp Tyr Ala Gly Glu Phe Val Leu Leu Arg Thr Trp Glu
        35                  40                  45


Ala Leu Ala Pro Ala Thr Pro Ala
    50                  55


<210>  14
<211>  7
<212>  PRT
<213>  artificial

<220>
<223>  amino acid sequence encoding the TEV cleavage site

<400>  14

Glu Asn Leu Tyr Phe Gln Gly
1               5

<210>  15
<211>  7
<212>  PRT
<213>  artificial

<220>
<223>  amino acid sequence encoding the TEV cleavage site n°2

<400>  15

Glu Asn Leu Tyr Phe Gln Ser
1               5

<210>  16
<211>  6
<212>  PRT
<213>  artificial

<220>
<223>  amino acid sequence encoding the enterokinase cleavage site

<400>  16

Asp Asp Asp Asp Lys Asp
1               5

<210>  17
<211>  1526
<212>  PRT
<213>  artificial

<220>
<223>  amino acid sequence of cMBP-TEV-hTERT

<400>  17

<210> 18
<211> 7480
<212> DNA
<213> artificial

<220>
<223> nucleotide sequence of the vector PGAPZ including cMBP-hTERT

<400> 18
agatcttttt tgtagaaatg tcttggtgtc ctcgtccaat caggtagcca tctctgaaat 60
atctggctcc gttgcaactc cgaacgacct gctggcaacg taaaattctc cggggtaaaa 120
cttaaatgtg gagtaatgga accagaaacg tctcttccct tctctctcct tccaccgccc 180
gttaccgtcc ctaggaaatt ttactctgct ggagagcttc ttctacggcc cccttgcagc 240
aatgctcttc ccagcattac gttgcgggta aaacggaggt cgtgtacccg acctagcagc 300
ccagggatgg aaaagtcccg gccgtcgctg gcaataatag cgggcggacg catgtcatga 360
gattattgga aaccaccaga atcgaatata aaaggcgaac acctttccca attttggttt 420
ctcctgaccc aaagacttta aatttaattt atttgtccct atttcaatca attgaacaac 480
tatttcgaag gtaccaagct tgccaccatg aaaatcgaag aaggtaaact ggtaatctgg 540
attaacggcg ataaaggcta taacggtctc gctgaagtcg gtaagaaatt cgagaaagat 600
accggaatta aagtcaccgt tgagcatccg gataaactgg aagagaaatt cccacaggtt 660
gcggcaactg gcgatggccc tgacattatc ttctgggcac acgaccgctt tggtggctac 720
gctcaatctg gcctgttggc tgaaatcacc ccggacaaag cgttccagga caagctgtat 780
ccgtttacct gggatgccgt acgttacaac ggcaagctga ttgcttaccc gatcgctgtt 840
gaagcgttat cgctgattta taacaaagat ctgctgccga acccgccaaa aacctgggaa 900
gagatcccgg cgctggataa agaactgaaa gcgaaaggta agagcgcgct gatgttcaac 960
ctgcaagaac cgtacttcac ctggccgctg attgctgctg acgggggtta tgcgttcaag 1020
tatgaaaacg gcaagtacga cattaaagac gtgggcgtgg ataacgctgg cgcgaaagcg 1080
ggtctgacct tcctggttga cctgattaaa aacaaacaca tgaatgcaga caccgattac 1140
tccatcgcag aagctgcctt taataaaggc gaaacagcga tgaccatcaa cggcccgtgg 1200
gcatggtcca acatcgacac cagcaaagtg aattatggtg taacggtact gccgaccttc 1260
aagggtcaac catccaaacc gttcgttggc gtgctgagcg caggtattaa cgccgccagt 1320
ccgaacaaag agctggcaaa agagttcctc gaaaactatc tgctgactga tgaaggtctg 1380
gaagcggtta ataaagacaa accgctgggt gccgtagcgc tgaagtctta cgaggaagag 1440
ttggcgaaag atccacgtat tgccgccacc atggaaaacg cccagaaagg tgaaatcatg 1500
ccgaacatcc cgcagatgtc cgctttctgg tatgccgtgc gtactgcggt gatcaacgcc 1560
gccagcggtc gtcagactgt cgatgaagcc ctgaaagacg cgcagactaa ttcgagctcg 1620
aacaacaaca acaataacaa taacaacaac gattacgatc caacgaccga aaacttatat 1680
tttcagggta tgccgcgcgc tccccgctgc cgagccgtgc gctccctgct gcgcagccac 1740
taccgcgagg tgctgccgct ggccacgttc gtgcggcgcc tggggcccca gggctggcgg 1800
ctggtgcagc gcggggaccc ggcggctttc cgcgcgctgg tggcccagtg cctggtgtgc 1860

gtgccctggg acgcacggcc gccccccgcc gccccctcct tccgccaggt gtcctgcctg 1920

aaggagctgg tggcccgagt gctgcagagg ctgtgcgagc gcggcgcgaa gaacgtgctg 1980

gccttcggct tcgcgctgct ggacggggcc cgcgggggcc cccccgaggc cttcaccacc 2040

agcgtgcgca gctacctgcc caacacggtg accgacgcac tgcgggggag cggggcgtgg 2100

gggctgctgc tgcgccgcgt gggcgacgac gtgctggttc acctgctggc acgctgcgcg 2160

ctctttgtgc tggtggctcc cagctgcgcc taccaggtgt gcgggccgcc gctgtaccag 2220

ctcggcgctg ccactcaggc ccggcccccg ccacacgcta gtggaccccg aaggcgtctg 2280

ggatgcgaac gggcctggaa ccatagcgtc agggaggccg gggtccccct gggcctgcca 2340

gccccgggtg cgaggaggcg cgggggcagt gccagccgaa gtctgccgtt gcccaagagg 2400

cccaggcgtg gcgctgcccc tgagccggag cggacgcccg ttgggcaggg gtcctgggcc 2460

cacccgggca ggacgcgtgg accgagtgac cgtggtttct gtgtggtgtc acctgccaga 2520

cccgccgaag aagccacctc tttggagggt gcgctctctg gcacgcgcca ctcccaccca 2580

tccgtgggcc gccagcacca cgcgggcccc ccatccacat cgcggccacc acgtccctgg 2640

gacacgcctt gtcccccggt gtacgccgag accaagcact tcctctactc ctcaggcgac 2700

aaggagcagc tgcggccctc cttcctactc agctctctga ggcccagcct gactggcgct 2760

cggaggctcg tggagaccat ctttctgggt tccaggccct ggatgccagg gactccccgc 2820

aggttgcccc gcctgcccca gcgctactgg caaatgcggc ccctgtttct ggagctgctt 2880

gggaaccacg cgcagtgccc ctacggggtg ctcctcaaga cgcactgccc gctgcgagct 2940

gcggtcaccc cagcagccgg tgtctgtgcc cgggagaagc cccagggctc tgtggcggcc 3000

cccgaggagg aggacacaga cccccgtcgc ctggtgcagc tgctccgcca gcacagcagc 3060

ccctggcagg tgtacggctt cgtgcgggcc tgcctgcgcc ggctggtgcc cccaggcctc 3120

tggggctcca ggcacaacga acgccgcttc ctcaggaaca ccaagaagtt catctccctg 3180

gggaagcatg ccaagctctc gctgcaggag ctgacgtgga agatgagcgt gcgggactgc 3240

gcttggctgc gcaggagccc aggggttggc tgtgttccgg ccgcagagca ccgtctgcgt 3300

gaggagatcc tggccaagtt cctgcactgg ctgatgagtg tgtacgtcgt cgagctgctc 3360

aggtctttct tttatgtcac ggagaccacg tttcaaaaga acaggctctt tttctaccgg 3420

aagagtgtct ggagcaagtt gcaaagcatt ggaatcagac agcacttgaa gagggtgcag 3480

ctgcgggagc tgtcggaagc agaggtcagg cagcatcggg aagccaggcc cgccctgctg 3540

acgtccagac tccgcttcat ccccaagcct gacgggctgc ggccgattgt gaacatggac 3600

tacgtcgtgg gagccagaac gttccgcaga gaaaagaggg ccgagcgtct cacctcgagg 3660

gtgaaggcac tgttcagcgt gctcaactac gagcgggcgc ggcgccccgg cctcctgggc 3720

gcctctgtgc tgggcctgga cgatatccac agggcctggc gcaccttcgt gctgcgtgtg 3780

cgggcccagg acccgccgcc tgagctgtac tttgtcaagg tggatgtgac gggcgcgtac 3840

gacaccatcc cccaggacag gctcacggag gtcatcgcca gcatcatcaa accccagaac 3900

acgtactgcg tgcgtcggta tgccgtggtc cagaaggccg cccatgggca cgtccgcaag 3960

gccttcaaga gccacgtctc taccttgaca gacctccagc cgtacatgcg acagttcgtg 4020

gctcacctgc aggagaccag cccgctgagg gatgccgtcg tcatcgagca gagctcctcc 4080

ctgaatgagg ccagcagtgg cctcttcgac gtcttcctac gcttcatgtg ccaccacgcc 4140

gtgcgcatca ggggcaagtc ctacgtccag tgccagggga tcccgcaggg ctccatcctc 4200

tccacgctgc tctgcagcct gtgctacggc gacatggaga acaagctgtt tgcggggatt 4260

cggcgggacg ggctgctcct gcgtttggtg gatgatttct tgttggtgac acctcacctc 4320

acccacgcga aaaccttcct caggaccctg gtccgaggtg tccctgagta tggctgcgtg 4380

gtgaacttgc ggaagacagt ggtgaacttc cctgtagaag acgaggccct gggtggcacg 4440

gcttttgttc agatgccggc ccacggccta ttcccctggt gcggcctgct gctggatacc 4500

cggaccctgg aggtgcagag cgactactcc agctatgccc ggacctccat cagagccagt 4560

ctcaccttca accgcggctt caaggctggg aggaacatgc gtcgcaaact ctttggggtc 4620

ttgcggctga agtgtcacag cctgtttctg gatttgcagg tgaacagcct ccagacggtg 4680

tgcaccaaca tctacaagat cctcctgctg caggcgtaca ggtttcacgc atgtgtgctg 4740

cagctcccat ttcatcagca agtttggaag aaccccacat ttttcctgcg cgtcatctct 4800

gacacggcct ccctctgcta ctccatcctg aaagccaaga acgcagggat gtcgctgggg 4860

gccaagggcg ccgccggccc tctgccctcc gaggccgtgc agtggctgtg ccaccaagca 4920

ttcctgctca agctgactcg acaccgtgtc acctacgtgc cactcctggg gtcactcagg 4980

acagcccaga cgcagctgag tcggaagctc ccggggacga cgctgactgc cctggaggcc 5040

gcagccaacc cggcactgcc ctcagacttc aagaccatcc tggactgagt cgaggtgcat 5100

gcggtgaatt caaaggccta cgtcgacgag ctcactagtc gcggccgctt tcgaatctag 5160

aacaaaaact catctcagaa gaggatctga atagcgccgt cgaccatcat catcatcatc 5220

attgagtttt agccttagac atgactgttc ctcagttcaa gttgggcact tacgagaaga 5280

ccggtcttgc tagattctaa tcaagaggat gtcagaatgc catttgcctg agagatgcag 5340

gcttcatttt tgatactttt ttatttgtaa cctatatagt ataggatttt ttttgtcatt 5400

ttgtttcttc tcgtacgagc ttgctcctga tcagcctatc tcgcagctga tgaatatctt 5460

gtggtagggg tttgggaaaa tcattcgagt ttgatgtttt tcttggtatt tcccactcct 5520

cttcagagta cagaagatta agtgagacct tcgtttgtgc ggatccccca cacaccatag 5580

cttcaaaatg tttctactcc ttttttactc ttccagattt tctcggactc cgcgcatcgc 5640

cgtaccactt caaaacaccc aagcacagca tactaaattt tccctctttc ttcctctagg 5700

gtgtcgttaa ttacccgtac taaaggtttg gaaaagaaaa aagagaccgc ctcgtttctt 5760

tttcttcgtc gaaaaaggca ataaaaattt ttatcacgtt tctttttctt gaaatttttt 5820

tttttagttt ttttctcttt cagtgacctc cattgatatt taagttaata aacggtcttc 5880

aatttctcaa gtttcagttt catttttctt gttctattac aacttttttt acttcttgtt 5940

cattagaaag aaagcatagc aatctaatct aagggcggtg ttgacaatta atcatcggca 6000

tagtatatcg gcatagtata atacgacaag gtgaggaact aaaccatggc caagttgacc 6060

agtgccgttc cggtgctcac cgcgcgcgac gtcgccggag cggtcgagtt ctggaccgac 6120

cggctcgggt tctcccggga cttcgtggag gacgacttcg ccggtgtggt ccgggacgac 6180

gtgaccctgt tcatcagcgc ggtccaggac caggtggtgc cggacaacac cctggcctgg 6240

gtgtgggtgc gcggcctgga cgagctgtac gccgagtggt cggaggtcgt gtccacgaac 6300

ttccgggacg cctccgggcc ggccatgacc gagatcggcg agcagccgtg ggggcgggag 6360

ttcgccctgc gcgacccggc cggcaactgc gtgcacttcg tggccgagga gcaggactga 6420

cacgtccgac ggcggcccac gggtcccagg cctcggagat ccgtccccct tttcctttgt 6480

cgatatcatg taattagtta tgtcacgctt acattcacgc cctcccccca catccgctct 6540

aaccgaaaag gaaggagtta gacaacctga agtctaggtc cctatttatt tttttatagt 6600

tatgttagta ttaagaacgt tatttatatt tcaaattttt cttttttttc tgtacagacg 6660

cgtgtacgca tgtaacatta tactgaaaac cttgcttgag aaggttttgg gacgctcgaa 6720

ggctttaatt tgcaagctgg agaccaacat gtgagcaaaa ggccagcaaa aggccaggaa 6780

ccgtaaaaag gccgcgttgc tggcgttttt ccataggctc cgcccccctg acgagcatca 6840

caaaaatcga cgctcaagtc agaggtggcg aaacccgaca ggactataaa gataccaggc 6900

gtttccccct ggaagctccc tcgtgcgctc tcctgttccg accctgccgc ttaccggata 6960

cctgtccgcc tttctccctt cgggaagcgt ggcgctttct caatgctcac gctgtaggta 7020

tctcagttcg gtgtaggtcg ttcgctccaa gctgggctgt gtgcacgaac cccccgttca 7080

gcccgaccgc tgcgccttat ccggtaacta tcgtcttgag tccaacccgg taagacacga 7140

cttatcgcca ctggcagcag ccactggtaa caggattagc agagcgaggt atgtaggcgg 7200

tgctacagag ttcttgaagt ggtggcctaa ctacggctac actagaagga cagtatttgg 7260

tatctgcgct ctgctgaagc cagttacctt cggaaaaaga gttggtagct cttgatccgg 7320

caaacaaacc accgctggta gcggtggttt ttttgtttgc aagcagcaga ttacgcgcag 7380

aaaaaaagga tctcaagaag atcctttgat cttttctacg gggtctgacg ctcagtggaa 7440

cgaaaactca cgttaaggga ttttggtcat gcatgagatc 7480

<210>  19
<211>  52
<212>  DNA
<213>  artificial

<220>
<223>  primer MBP-F

<400>  19
atgcaattcg aaggtaccaa gcttgccacc atgaaaatcg aagaaggtaa ac          52


<210>  20
<211>  35
<212>  DNA
<213>  artificial

<220>
<223>  primer MBP-R

<400>  20
tcgttggatc gtaatcgttg ttgttattgt tattg          35


<210>  21
<211>  46
<212>  DNA
<213>  artificial

<220>
<223>  primer hTERT-F

<400>  21
ccgaaaactt atattttcag ggtatgccgc gcgctccccg ctgccg          46


<210>  22
<211>  47
<212>  DNA
<213>  artificial

<220>
<223>  primer hTERT-R

<400>  22
cttcaagacc atcctggact gagtcgagcc gcggcggccg catgcaa          47